# EUROPEAN PATENT SPECIFICATION

(11) **EP 2 929 354 B1**
(45) Date of publication and mention of the grant of the patent: **18.04.2018**
(21) Application number: 13799310.1
(22) Date of filing: 04.12.2013
(51) Int. Cl.: G01N 33/68

(54) **BIOMARKERS IN THE SELECTION OF THERAPY OF HEART FAILURE**
BIOMARKER FÜR DIE AUSWAHL EINER THERAPIE FÜR HERZVERSAGEN
BIOMARQUEURS DANS LA SÉLECTION DU TRAITEMENT D'UNE INSUFFISANCE CARDIAQUE

(30) Priority: 04.12.2012 EP 12195491
(43) Date of publication of application: 14.10.2015
(73) Proprietor: Roche Diagnostics GmbH, 68305 Mannheim (DE); F. Hoffmann-La Roche AG, 4070 Basel (CH)
(72) Inventor: ZAUGG, Christian, CH-4310 Rheinfelden (CH); BLOCK, Dirk, 83673 Bichl (DE); WIENHUES-THELEN, Ursula-Henrike, 82152 Krailling (DE); BRUNNER, Hans-Peter, CH-4142 Münchenstein (CH); KRAUSE, Friedemann, D-82377 Penzberg (DE); MODEL, Fabian, D-79618 Rheinfelden (DE); ROLNY, Vincent, D-80337 München (DE)
(74) Representative: Herzog, Fiesser & Partner Patentanwälte PartG mbB
(86) International application number: PCT/EP2013/075491
(87) International publication number: WO 2014/086833

(56) References cited:
- WO-A1-2008/089994
- WO-A1-2009/138451
- WO-A1-2009/138451
- WO-A1-2010/124821
- WO-A1-2012/025355
- US-A1- 2009 155 827
- US-A1- 2009 155 827
- IRIS Z. JAFFE ET AL: "Placental growth factor mediates aldosterone-dependent vascular injury in mice", JOURNAL OF CLINICAL INVESTIGATION, vol. 120, no. 11, 1 November 2010 (2010-11-01), pages 3891-3900, XP055318027, US ISSN: 0021-9738, DOI: 10.1172/JCI40205
- IRIS Z. JAFFE ET AL: "Placental growth factor mediates aldosterone-dependent vascular injury in mice", JOURNAL OF CLINICAL INVESTIGATION, vol. 120, no. 11, 1 November 2010 (2010-11-01), pages 3891-3900, XP055318027, US ISSN: 0021-9738, DOI: 10.1172/JCI40205

## Description

For the purpose of the present description, the word "invention" should be read as "disclosure" until the chapter "THE INVENTION" below.

The present invention relates to a method for identifying a subject being eligible to the administration of at least one medicament selected from the group consisting of a beta adrenergic receptor blocker (beta blocker), an aldosterone antagonist, a diuretic, and an inhibitor of the angiotensin converting enzyme (ACE) or an angiotensin receptor blocker (ARB), the latter two collectively referred to as inhibitors of the renin-angiotensin system. The method is based on the determination of the amount of at least one biomarker selected from the group consisting of GDF-15 (Growth Differentiation Factor 15), endostatin, mimecan, IGFBP7 (IGF binding protein 7), a cardiac Troponin, a BNP-type peptide, uric acid, Galectin-3 (Gal-3), soluble ST2 (sST2), P1GF, sFlt-1, P1NP, Cystatin C, Prealbumin, and Transferrin in a sample from a subject suffering from heart failure. Further, the method comprises the step of comparing the, thus, determined amount with a reference amount. Further envisaged by the present invention are kits and devices adapted to carry out the method of the present invention. The present invention also relates to a system for identifying a subject being eligible to the administration of at least one medicament as disclosed herein and to reagents and kits used in performing the methods as disclosed herein.

An aim of modem medicine is to provide personalized or individualized treatment regimens. Those are treatment regimens which take into account a patient's individual needs or risks. Personalized or individual treatment regimens shall be even taken into account for measures where it is required to decide on potential treatment regimens.

Heart failure (HF) is a major and growing public health problem. It is estimated that approximately 5 million patients in the USA have HF, more than 500 000 patients are diagnosed with HF for the first time each year, and more than 250.000 patients in the US die each year of HF as a primary cause. Heart failure (HF) is one of the main causes of morbidity and mortality in developed countries. Because of aging of the population and greater longevity of patients with cardiovascular disease incidence and prevalence of HF are increasing.

Heart failure is a complex clinical syndrome that can result from any structural or functional cardiac disorder that impairs the ability of the ventricle to fill with or eject blood and to ensure the body's metabolic needs for supply with blood/oxygen. In such cases, the body tries to compensate lack of supply by structural changes of the myocardium (e.g. hypertrophy eventually leading to fibrosis, apoptosis, necrosis) and neurohumoral stimulation (activation of sympathetic nervous system and renin angiotensin aldosterone system) aiming at maintaining the required supply. HF is classified into various degrees of severity.

One classification is the so-called NYHA (New York Heart Association) classification. Heart failure patients are classified NYHA classes I, II, III and IV or American College of Cardiology and the American Heart Association (ACC/AHA) stages A, B, C, and D. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;e1-e82). 4 stages A, B, C and D are defined. A patient having heart failure stage B, C or D has already experienced structural and functional changes to the heart. He will not be able to fully restore his health, and is in need of a therapeutical treatment.

Medication and treatment guidelines for acute and chronic heart failure are described in the ESC Guidelines for the diagnosis and treatment of acute and chronic heart failure (European Heart Journal (2008) 29, 2388-2442). Although available treatment options can reduce morbidity and mortality in patients with heart failure (HF), the relative number of eligible patients receiving these treatments remains unsatisfactorily low (O'Donoghue M. & Braunwald E., Nat. Rev. Cardiol. 2010; 7: 13-20). Furthermore, in patients eligible for treatment, therapy has been primarily guided and adjusted by underlying disease as well as signs and symptoms of HF to maximal tolerability of drugs (e.g. by NYHA stages, ACC/AHA stages, or congestion scores).

Measurement of natriuretic peptide markers, such as B-type natriuretic peptide (BNP), or its amino-terminal fragment N-terminal proBNP (NT-proBNP), has emerged as an important tool for guiding therapy in patients with systolic HF (O'Donoghue M. & Braunwald E., Nat. Rev. Cardiol. 2010; 7: 13-20). BNP/NT-proBNP guided HF therapy can identify patients in need of therapy intensification and indicate the timing for such intensification. However, BNP/NT-proBNP guided HF therapy does not indicate what kind of drug therapies patients will likely benefit from.

For example, the beneficial effects of spironolactone on the treatment of cardiovascular diseases in patients with severe symptomatic HF of NYHA class II, III or IV are well known, however these drugs are also known for severe side-effects (e.g. hyperkalemia, arrhythmias, sudden death, hypotension, impotence, muscle weakness, gynecomastia, and gastritis) Williams et al. 2006 Clin. Cardiol. 29, 149-153. Especially the risk to develop hyperkalemia is a threat (D.N. Juurlink et.al. NEJM 2004; 351 543).

In other words, underlying diseases, clinical judgment and BNP/NT-proBNP guided HF therapy alone do not provide sufficient information about the selection of treatment(s) or therapy intensification. New biomarkers are needed in addition and in particular those who can predict response to therapy or help to select an appropriate therapy, to intensify a particular therapy, or conversely to avoid a particular therapy or intensification thereof.

Therefore, there is need for biomarkers that would allow for identifying subjects which would benefit from certain heart failure therapies or conversely would experience harm form certain heart failure therapies.

The IGFBP system plays an important role in cell growth and differentiation. IGF binding protein 7 (=IGFBP-7) is a 30-kDa modular glycoprotein known to be secreted by endothelial cells, vascular smooth muscle cells, fibroblasts, and epithelial cells (Ono, Y., et al., Biochem Biophys Res Comm 202 (1994) 1490-1496). In the literature this molecule has also been denominated as FSTL2; IBP 7; IGF binding protein related protein I; IGFBP 7; IGFBP 7v; IGFBP rP1; IGFBP7; IGFBPRP1; insulin like growth factor binding protein 7; insulin like growth factor binding protein 7 precursor; MAC25; MAC25 protein; PGI2 stimulating factor; and PSF or Prostacyclin stimulating factor. Low levels of IGFB-7 were detected in random human sera and increased serum levels have been seen in association with insulin-resistance (Lopez-Bermejo, A., et al., J. Clinical Endocrinology and Metabolism 88 (2003) 3401-3408, Lopez-Bermejo, A., et al., Diabetes 55 (2006) 2333-2339).

US20100285491 discloses the use of IGFBP-7 in the assessment of heart failure. It further discloses that the marker IGFBP-7 can be used for selecting a treatment regimen for a patient suffering from HF. In addition it is claimed that in the follow-up of HF patients an elevated level of IGFBP-7 is a positive indicator for an effective treatment of HF.

WO2008/015254 discloses a GDF-15 detection based method of identifying a subject being eligible to a therapy of heart failure, preferably a drug based therapy using a medicament like an aldosterone antagonist including spironolacton. Preferably, there is additional detection of TnT or NTproBNP. Moreover, preferably an amount of GDF- 15 (preferably 1200 pg/ml) larger than the reference amount is indicative for a subject eligible to a therapy of heart failure. However, the document does not disclose which drug therapy should be considered or if a medication adjustment or therapy intensification should be considered.

Mimecan (also referred to as osteoglycin) is a multifunctional component of the extracellular matrix. Mimecan has been shown to be involved in regulating collagen fibrillogenesis, a process essential in development, tissue repair, and metastasis (Tasheva et al., MoI. Vis. 8 (2002) 407-415). It plays a role in bone formation in conjunction with TGF-beta-1 or TGF-beta-2. Transcriptome analyses in rat and human heart tissue revealed a high correlation of mimecan with left ventricular mass as well as with extracellular remodelling in dilatative cardiomyopathy (Petretto, E. et al., Nature Genetics 40 (2008) 546-552.

WO2011/01226 discloses that the use of mimecan in a bodily fluid sample derived from an individual for assessing heart failure. It further relates to the use of the marker Mimecan in selecting a treatment regimen for a patient suffering from HF. In addition it is disclosed that in the follow-up of HF patients an elevated level of Mimecan is a positive indicator for an effective treatment of HF. However, the document does not disclose which drug therapy should be considered or if a medication adjustment or therapy intensification should be considered.

Endostatin was originally isolated from murine hemangioendothelioma as a 20 kDA proteolytic fragment of type XVIII collagen (O'Reilly, M.S. et al., Cell 88 (1997) 277-285). Collagens represent a family of extracellular matrix proteins with a characteristic triple-helical conformation forming supra-molecular aggregates that play a dominant role in maintaining tissue structural integrity. Excessive collagen deposition leads to fibrosis disrupting the normal functioning of surrounding tissues. Endostatin is released from the alpha 1 chain of collagen XVIII by action of various proteolytic enzymes (Ortega, N. and Werb, Z., Journal of Cell Science 115 (2002) 4201-4214). Endostatin is a potent inhibitor of angiogenesis and blood vessel growth. However, the document does not disclose which drug therapy should be considered or if a medication adjustment or therapy intensification should be considered.

WO2010/124821 discloses that Endostatin is measured in order to assess heart failure. It further relates to relates to the use of the marker Endostatin in selecting a treatment regimen for a patient suffering from HF. In addition it is claimed that in the follow-up of HF patients an elevated level of Endostatin is a positive indicator for an effective treatment of HF.

Growth-differentiation factor-15 (GDF-15) is a member of the transforming growth factor-β cytokine superfamily and was first identified as macrophage-inhibitory cytokine-1 (MIC-1), and later also named placental transforming growth factor-β (Bootcov 1997, Proc Natl Acad Sci 94:11514-11519; Tan 2000, Proc Natl Acad Sci 97:109-114). GDF-15 has been described as a strong predictor of cardiovascular events and an indicator for cardiovascular complications, (Brown, D. A. et al., 2002 The Lancet, 359: 2159-2163; US2003/0232385; Kempf 2006, Circ Res 98: 351-360;) Kempf et al. showed that circulating levels of GDF-15 are related to severity of CHF and predict the risk of death in patients with chronic heart failure (Clinical Chemistry 53:2; 284-291 (2007); Am Coll Cardiol, 2007; 50:1054-1060).

WO2012/025355 discloses not only methods for diagnosis or monitoring functional and/or structural abnormalities of the heart preceding HF but also the guidance for selecting an appropriate drug to treat such conditions. The marker can be IGFBP7. WO2010/007041 discloses a GDF-15, NT-proANP, NT-proBNP, and cardiac troponin detection based method of monitoring an apparently stable subject suffering from heart failure. Moreover it discloses a method of diagnosing and/or deciding which therapy/medication is to be applied in an apparently stable subject suffering from heart failure and undergoing a change in its physiological state.

WO2009/047283 describes a method of diagnosing which treatment or combinations of treatments is to be applied in the remodeling process of a subject after myocardial infarction by determining BNP, cTnT and a least one inflammatory marker osteopontin (OPN), GDF-15, CRP. It describes a level of osteopontin >/= 500 pg/ml as indicative for ACE-inhibitors, angiotensin receptor antagonists, and aldosterone antagonists.

Kubo et al. 2011 (Circulation J, 75, 919-926) discloses a BNP and Troponin based detection based risk prediction for clinical deterioration in patients suffering from hypertrophic cardiomyopathy. Many of the assessed patients suffered from heart failure. It is speculated that combined measurement of the two markers may be useful for monitoring patients with hypertrophic cardiomyopathy.

Fonarow et al. 2008 (Am J Cardiol, 101, 231-237) discloses a BNP and Troponin detection based mortality risk prediction in patients suffering from heart failure.

Mentz et al. 2011 (Circ J, 75(9):2031-7) discloses a NTproBNP detection based method for therapy guidance and monitoring in a heart failure patient, wherein the therapy is selected from treatment with diuretics, ACE inhibitor, beta blocker, spironolactone, nitrates or digoxin.

Böhm et al. 2011 (Clin Res Cardiol, 100:973-981) reviews NTproBNP detection based method for therapy guidance and monitoring in a heart failure patient. It also mentions the possibility of combining BNP with troponin for guiding heart failure therapy.

US 2009/155827 discloses a method comprising the determination of P1GF and sFlt-1 in a sample for diagnosis, risk stratification and/or monitoring of a vascular disease with atherosclerotic etiology, in particular a coronary heart disease such a unstable angina pectoris or myocardial infarction, and/or for estimation of the probability of developing such a disease, as well as for identification of a patient supposed to benefit from a therapy by agents reducing the risk for a cardiovascular disease.

WO 2009/138451 discloses a method for diagnosing if a subject suffering from dilated cardiomyopathy is suffering from ischemic or non-ischemic dilated cardiomyopathy. Amongst others, an angiogenic marker is determined, which can be P1GF and/or sFlt-1.

IRIS Z. JAFFE ET AL., JOURNAL OF CLINICAL INVESTIGATION, vol. 120, no. 11, Nov. 2010, p.3891-3900 teaches that P1GF mediates aldosterone-dependent vascular injury in mice. The findings suggest a non-renal mechanism for the vascular protective effects of aldosterone antagonists in humans and support targeting the vascular aldosterone/MR/PGF/Flt1 pathway as a therapeutic strategy for ischemic cardiovascular disease.

In the context of the studies underlying the present invention, it was advantageously shown that the determination of GDF-15, endostatin, mimecan, IGFBP7, a cardiac Troponin, a BNP-type peptide, uric acid, Galectin-3, soluble ST2, PlGF, sFlt-1, P1NP, Cystatin C, Prealbumin and/or Transferrin allows for identifying subjects which would benefit from certain heart failure therapies. It was shown that only certain groups of patients would benefit from the administration of e.g. beta blockers and aldosterone antagonist, wherein other groups would not benefit from the administration. By determining the amounts of the aforementioned biomarkers it is possible to differentiate between subjects who will benefit from the therapy and subjects who will not benefit from the therapy or who would even experience side-effects or harm from the therapy due to unnecessary intensification / dose uptitration in the absence of a beneficial effect.

The technical problem underlying the present invention can be seen as the provision of means and methods for complying with the aforementioned needs.

The technical problem is solved by the embodiments characterized in the claims and herein below.

Accordingly, the present invention relates to a method for identifying a subject being eligible to the administration of at least one medicament for the therapy of heart failure, comprising
a) determining the amount of at least one biomarker in a sample from a subject suffering from heart failure, and
b) comparing the amount (or amounts) of the at least one biomarker as determined in step a) with a reference amount (or with reference amounts), whereby a subject being eligible to the administration of said at least one medicament is identified.

Preferably, said at least one medicament is selected from the group consisting of a beta blocker, an aldosterone antagonist, a diuretic, and an inhibitor of the renin-angiotensin system.

Preferably, said at least one biomarker is selected from the group consisting of IGFBP7 (IGF binding protein 7), mimecan, osteopontin, endostatin, sFlt-1 (soluble fins-like tyrosine kinase 1), PlGF (Placental Growth Factor), a cardiac Troponin, a BNP-type peptide, uric acid, GDF-15 (Growth Differentiation Factor 15), sST2 (soluble ST2), Gal3 (Galectin-3), P1NP (Procollagen Type 1 N-Terminal Propeptide), Cystatin C, Prealbumin and Transferrin.

Accordingly, the present invention relates to a method for identifying a subject being eligible to the administration of at least one medicament selected from the group consisting of a beta blocker, an aldosterone antagonist, a diuretic, and an inhibitor of the renin-angiotensin system, comprising
a) determining the amount of at least one biomarker selected from the group consisting of IGFBP7 (IGF binding protein 7), mimecan, osteopontin, endostatin, sFlt-1 (soluble fins-like tyrosine kinase 1), PlGF (Placental Growth Factor), a cardiac Troponin, a BNP-type peptide, uric acid, GDF-15 (Growth Differentiation Factor 15), sST2 (soluble ST2), Gal3 (Galectin-3), P1NP (Procollagen Type 1 N-Terminal Propeptide), Cystatin C, Prealbumin and Transferrin and in a sample from a subject suffering from heart failure, and
b) comparing the amount (or amounts) as determined in step a) with a reference amount (or with reference amounts), whereby a subject being eligible to the administration of said at least one medicament is identified.

In a preferred embodiment of the method of the present invention, the at least one medicament is an aldosterone antagonist. In this case, the at least one biomarker is selected from IGFBP7, endostatin, uric acid, a BNP-type peptide, a cardiac Troponin, sFlt-1, PlGF, sST2, Gal-3, and Cystatin C. Preferably, the amount of only one of the aforementioned biomarkers is determined. However, it is also contemplated to determine the amount of more than one biomarker in combination. Preferred combinations are s-Flt-1 and IGFBP7; s-Flt-1 and a cardiac Troponin; s-Flt-1 and Gal-3; a cardiac Troponin and Gal-3; a cardiac Troponin and IGFBP7; IGPBP7 and Gal-3; and combinations of these markers with a BNP-type peptide. A particularly preferred combination is IGFBP7 and mimecan. A further preferred combination is a combination of PlGF and sFlt-1 Further, it is envisaged to calculate the ratio of the amount of PlGF to the amount of sFlt-1 (or the ratio of the amount to sFlt-1 to PlGF). In this case, the calculated ratio is compared to a reference ratio.

In another preferred embodiment of the method of the present invention, the at least one medicament is a beta blocker. In this case, said at least one biomarker is, preferably, selected from the group consisting of IGFBP7, GDF-15, endostatin, mimecan, a BNP-type peptide, a cardiac Troponin, osteopontin, sFlt-1 and P1NP. Preferably, the amount of only one biomarker is determined. However, it is also contemplated to determine the amount of more than one biomarker in combination. Preferred combinations are GDF-15 and endostatin; GDF-15 and a cardiac Troponin; GDF-15 and mimecan; GDF-15 and sST2; endostatin and a cardiac Troponin; endostatin and mimecan; endostatin and IGFBP7; mimecan and a cardiac Troponin; mimecan and IGFBP7; a cardiac Troponin and IGFBP7. A particularly preferred combination is endostatin and sFlt-1. A further preferred combination is endostatin and PlGF.

In another preferred embodiment of the method of the present invention, the at least one medicament is an inhibitor of the renin-angiotensin system. In this case, said at least one biomarker is, preferably, osteopontin, GDF-15, a BNP-type peptide, sFlt-1, a cardiac Troponin, Gal-3, uric acid, or IGFBP7. Preferably, the amount of one biomarker is determined. However, it is also contemplated to determine the amount of more than one biomarker in combination. Preferred combinations are: a cardiac Troponin and Gal-3, a cardiac Troponin and uric acid, and Gal-3 and uric acid. Further preferred combinations are: osteopontin and sFlt-1, sFlt-1 and a cardiac Troponin, and osteopontin and a cardiac Troponin.

In another preferred embodiment of the method of the present invention, the at least one medicament is a diuretic. In this case, said at least one biomarker is, preferably, selected from the group consisting of GDF-15, endostatin, mimecan, a BNP-type peptide, uric acid, osteopontin, IGFBP-7, Gal-3, transferrin or prealbumin. Preferably, the amount of only one biomarker is determined. However, it is also contemplated to determine the amount of more than one biomarker in combination. Preferred combinations are GDF-15 and endostatin; GDF-15 and a BNP-type peptide; GDF-15 and mimecan; GDF-15 and Gal-3; endostatin and a BNP-type peptide; endostatin and mimecan; endostatin and Gal-3; mimecan and a BNP-type peptide; mimecan and Gal-3; a BNP-type peptide and Gal-3; BNP-type peptide and uric acid; a BNP-type peptide and IGFBP-7; a BNP-type peptide and osteopontin. In particular, a combination of uric acid and GDF-15 is envisaged.

Accordingly, the present invention, in particular, envisages the following methods:
A method is contemplated for identifying a subject being eligible to the administration (i.e. to the initial administration or intensification, in particular the administration at a higher dosage) of an aldosterone antagonist, comprising
   a) determining the amount of at least one biomarker selected from the group consisting of IGFBP7, endostatin, uric acid, a BNP-type peptide, a cardiac Troponin, sFlt-1, P1GF, sST-2, Gal-3, and Cystatin C in a sample from a subject suffering from heart failure, and
   b) comparing the amount (or amounts) as determined in step a) with a reference amount (or with reference amounts), whereby a subject being eligible to the administration of said aldosterone antagonist is identified.

In an embodiment, the amounts of PlGF and sFlt-1 are determined in step a), a ratio of the amount of PlGF to the amount of sFlt-1 (or the amount of sFlt-1 to PlGF) is calculated in a further step a1), and the thus compared ratio is compared to a reference ratio in step b).

Accordingly, the method may comprise the steps of:
a) determining the amounts of PlGF and sFlt-1 in a sample from a subject suffering from heart failure,
a1) calculating a ratio of the amount of PlGF to the amount of sFlt-1 (or vice versa), and
b) comparing the ratio calculated in step a1) with a reference ratio, whereby a subject being eligible to the administration of said aldosterone antagonist is identified.

Also contemplated is a method for identifying a subject being eligible to the administration (i.e. to the initial administration or intensification of administration, in particular the administration at a higher dosage) of a beta blocker, comprising
a) determining the amount of at least one biomarker selected from the group consisting of IGFBP7, GDF-15, endostatin, mimecan, a BNP-type peptide, a cardiac Troponin, osteopontin, sFlt-1 and P1NP in a sample from a subject suffering from heart failure, and
b) comparing the amount (or amounts) as determined in step a) with a reference amount (or with reference amounts), whereby a subject being eligible to the administration of said beta blocker is identified.

Further envisaged is a method for identifying a subject being eligible to the administration (i.e. to the initial administration or intensification of administration, in particular the administration at a higher dosage) of an inhibitor of the renin-angiotensin system, comprising
a) determining the amount of the biomarker selected from the group consisting of osteopontin, GDF-15, a BNP-type peptide, sFlt-1, a cardiac Troponin, Gal-3, uric acid, and IGFBP7 in a sample from a subject suffering from heart failure, and
b) comparing the amount (or amounts) as determined in step a) with a reference amount (or with reference amounts), whereby a subject being eligible to the administration of said an inhibitor of the renin-angiotensin system is identified.

The present invention also contemplates a method for identifying a subject being eligible to the administration (i.e. to the initial administration or intensification of administration) of a diuretic, comprising
a) determining the amount of at least one biomarker selected from the group consisting of IGFBP7, a cardiac Troponin, endostatin, mimecan, a BNP-type peptide, uric acid, osteopontin, Gal-3, prealbumin and transferrin in a sample from a subject suffering from heart failure, and
b) comparing the amount (or amounts) as determined in step a) with a reference amount (or with reference amounts), whereby a subject being eligible to the administration of said diuretic is identified.

Preferably, a subject is identified as being eligible the administration of at said at least one medicament, by carrying out the further step c) of identifying of a subject being eligible to the administration of said at least one medicament.

In an embodiment, the amount of the at least one biomarker is measured by contacting the sample with an agent that specifically binds to the respective marker, thereby forming a complex between the agent and said marker, detecting the amount of complex formed, and thereby measuring the amount of said marker. If the biomarker is uric acid, the level of said biomarker may be measured by contacting the sample with an enzyme or compound that allows for the conversion of said biomarker, e.g. that allows for the oxidation of uric acid. The enzyme may be an uricase (EC 1.7.3.3) which catalyzes the oxidation of uric acid to 5-hydroxyisourate. The compound may be phosphotungstic acid.

The following biomarker-medicament combinations are the most preferred combinations:
In a preferred embodiment of the methods, uses, kits, systems and devices of the present invention, the biomarker is IGFBP-7, and the medicament is a beta blocker.

In another preferred embodiment, the biomarker is mimecan, and the medicament is a beta blocker.

In another preferred embodiment, the biomarker is osteopontin, and the medicament is an inhibitor of the renin-angiotensin system. It is also envisaged that the medicament is a beta blocker.

In another preferred embodiment, the biomarker is Endostatin, and the medicament is an aldosterone antagonist.

In another preferred embodiment, the biomarker is sFlt-1, and the medicament is an aldosterone antagonist. It is also preferred that the biomarker is sFlt-1, and that the medicament is an inhibitor of the renin-angiotensin system. Further, it is envisaged that the medicament is a beta blocker.

In another preferred embodiment, the biomarker is PlGF, and the medicament is an aldosterone antagonist.

In another preferred embodiment, the biomarker is a cardiac Troponin, and the medicament is an inhibitor of the renin-angiotensin system

In another preferred embodiment, the biomarker is a BNP-type peptide, and the medicament is an inhibitor of the renin-angiotensin system. It is also preferred that biomarker is a BNP-type peptide, and that the medicament is a beta blocker.

In another preferred embodiment, the biomarker is uric acid, and the medicament is a diuretic. It is also preferred that the biomarker is uric acid, and that the medicament is an inhibitor of the renin-angiotensin system.

In another preferred embodiment, the biomarker is GDF-15, and the medicament is a diuretic. It is also preferred that the biomarker is GDF-15, and that the medicament is an inhibitor of the renin-angiotensin system.

In another preferred embodiment, the biomarker is sST2, and the medicament is an aldosterone antagonist. It is also preferred that biomarker is sST2, and that the medicament is a beta blocker.

In another preferred embodiment, the biomarker is P1NP, and the medicament is a beta blocker.

In another preferred embodiment, the biomarker is Cystatin C, and the medicament is an aldosterone antagonist.

In another preferred embodiment, the biomarker is prealbumin, and the medicament is a diuretic such as a loop diuretic.

In another preferred embodiment, the biomarker is IGFBP7, and the medicament is an inhibitor of the renin-angiotensin system.

Further preferred biomarker/medicament combinations are disclosed in the Examples.

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or assessment based on said comparison in step (b).

According, the present invention also preferably relates to a system identifying of a subject being eligible to the administration of at least one medicament selected from the group consisting of a beta blocker, an aldosterone antagonist, a diuretic, and an inhibitor of the renin-angiotensin system, comprising
a) an analyzer unit configured to contact, in vitro, a portion of a sample from the subject with a ligand (or ligands if the amount of more than one biomarker is determined) comprising specific binding affinity for at least one biomarker selected from the group consisting of GDF-15 (Growth Differentiation Factor 15), endostatin, mimecan, IGFBP7 (IGF binding protein 7), a cardiac Troponin, a BNP-type peptide, uric acid, Galectin-3 (Gal-3), soluble ST2 (sST2), PlGF, sFlt-1, P1NP, Cystatin C, Prealbumin, and Transferrin,
b) an analyzer unit configured to detect a signal from the portion of the sample from the subject contacted with the ligand (or ligands),
c) a computing device having a processor and in operable communication with said analysis units, and
d) a non-transient machine readable media including a plurality of instruction executable by a the processor, the instructions, when executed calculate an amount of the at least one biomarker, and compare the amount of the at least one biomarker with a reference amount (or reference amounts if the amount of more than one marker is determined), thereby identifying of a subject being eligible to the administration of at least one medicament.

The term "identifying" as used herein means assessing whether a subject will be eligible to the administration of said at least one medicament, or not. It is to be understood that a subject who is eligible to the administration of the at least one medicament will benefit from the administration of the at least one medicament, whereas a subject who is not eligible to the administration of said at least one medicament may experience adverse side-effects or harm from the administration of the at least one medicament. In particular, a subject benefits from the administration of said at least one medicament, if the administration of said at least one medicament reduces the risk of mortality of said subject and/or reduces the risk of hospitalization of said subject, in particular within a window period of 18 months or 3 years after the sample has been obtained. Preferably, the aforementioned risk (or risks) is (are) reduced by 5%, more preferably by 10%, even more preferably by 15% and, most preferably by 20%. Preferably, the hospitalization referred to herein shall be due to heart failure.

In contrast, a subject who is not eligible to the administration of the at least one medicament will not benefit (in particular will not benefit significantly) from the administration of the at least one medicament. In particular, a subject does not benefit from the administration of said at least one medicament, if the administration of said at least one medicament does not reduce (in particular, does not reduce significantly) the risk of mortality of said subject and/or does not reduce (in particular, does not reduce significantly) the risk of hospitalization of said subject and/or increases the risk of unwanted effects, in particular within a window period of 18 months or 3 years after the sample has been obtained. In this case, unnecessary health care costs can be avoided, if the medicament is not administered. Further, adverse side effects that may result from the administration can be avoided.

Thus, by identifying a subject who is eligible to the administration of said at least one medicament, it can be assessed whether a subject will benefit from the administration (i.e. from the initial administration or intensification of administration, in particular the administration at a higher dosage), or not. Accordingly, the present invention also relates to a method of identifying a subject who will benefit from the administration of said at least one medicament, based on the steps set forth herein elsewhere.

As will be understood by those skilled in the art, the assessment whether a subject is eligible to the administration of said at least one medicament is usually not intended to be correct for 100% of the subjects to be assessed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention.

In context of the present invention a subject shall be identified who is eligible to the administration of at least one medicament selected from the group consisting of a beta blocker, an aldosterone antagonist, a diuretic, and an inhibitor of the renin-angiotensin system.

With "administration" in the context of the method of the present invention it is meant that (i) the administration of said at least one medicament shall be initiated (and, thus, shall be added to the therapy) or that (ii) said at least one medicament shall be administered at a higher dosage (and, thus, shall be uptitrated).

The term "dosage" as used herein preferably refers to the daily dosage.

In the first case (i), the subject shall not have taken said at least one medicament previously. Thus, the said at least one medicament shall not have been administered to said subject before the sample to be tested has been obtained. Preferably, said at least one medicament has not been administered to the subject for at least three months, more preferably, for at least six months prior to obtaining the sample to be tested.

In the latter case (ii), the subject shall be treated already with said at least one medicament (prior to obtaining the sample). Preferably, the subject has been treated with said at least one medicament for at least two weeks, more preferably, for at least two months, even more preferably for at least six months, and, most preferably, for at least one year prior to obtaining the sample to be tested. Preferably, the dosage of said at least one medicament was not altered in this period.

It is particularly preferred that the subject to be tested is already treated with said at least one medicament and that it is assessed whether the subject is eligible (or not) to the administration of said medicament at a higher dosage, i.e. whether the therapy shall be continued with a higher dosage of said at least one medicament. Thus, it can be assessed whether the dosage of said at least one medicament shall be uptitrated.

In summary, a subject who is eligible to the administration of said at least one medicament is eligible to the initiation of administration of said at least one medicament or to the administration of said at least one medicament at a higher dosage, whereas a subject who is not eligible to the administration of said at least one medicament is not eligible to the initiation of the administration of said at least one medicament (in the case that the subject was not treated with said medicament prior to obtaining the sample to be tested) or is not eligible to the administration of said medicament at a higher dosage (in the case that the subject was treated with said medicament prior to obtaining the sample to be tested). Accordingly, if a subject is eligible to the administration of the medicament, the administration of said medicament can be initiated, or the dosage of said medicament can be increased. If a subject, however, is not eligible to the administration of said at least one medicament, administration of said medicament shall not be initiated or shall not be uptitrated a higher dosage. Accordingly, if the subject was treated with said medicament prior to obtaining the sample to be tested, the therapy shall be continued without altering the dosage, in particular, without increasing the dosage, if the subject is not eligible to the administration of said medicament. Also preferably, a subject who is not eligible to the administration of a medicament may receive a lower dose of said medicament.

The medicaments referred to in the context of the present invention are well known in the art, e.g. described in see e.g. Heart Disease, 2008, 8th Edition, Eds. Braunwald, Elsevier Sounders, chapter 24 or the ESC Guidelines for the diagnosis and treatment of acute and chronic heart failure (European Heart Journal (2008) 29, 2388-2442).

Beta blockers (frequently also referred to as beta adrenergic blockers) block the action of endogenous catecholamines epinephrine (adrenaline) and norepinephrine (noradrenaline) in particular, on β-adrenergic receptors. Preferably, the beta blocker is selected from the group consisting of cebutolol, alprenolol, atenolol, betaxolol, bisoprolol, bupranolol, carazolol, carteolol, carvedilol, celiprolol, metipranolol, metoprolol, nadolol, nebivolol, oxprenolol, penbutolol, pindolol, pro-panolol, sotalol, tanilolol, and timolol. The most preferred beta blockers areatenolol, carvedilol, metoprolol, and bisoprolol.

Aldosterone antagonists belong to a class of diuretic drugs which antagonize the action of aldosterone at mineralocorticoid receptors. They are often used as adjunctive therapy, in combination with other drugs, for the management of chronic heart failure. Preferably, the aldosterone antagonist is selected from the group consisting of eplerone, spironolactone, canrenone, mexrenone, prorenone. A particularly preferred aldosterone antagonist is spironolactone (7α-acetylthio-3-oxo-17α-pregn-4-ene-21,17-carbolactone) or eplerenone.

Diuretics are diuretics that increase the elimination of sodium and water via urine. A particularly preferred diuretic is a loop diuretic or a thiazide diuretic.

Preferably, loop diuretics are used due to their efficacy and rapid onset of action. They act on the ascending loop of Henle in the kidney. Preferably, the loop diuretic is selected from the group of: furosemide, azosemide, bumetanide, piretanide, torasemide, ethacrynic acid, etozolin. In particular, the loop diuretic is furosemide.

Preferably, the thiazide diuretic is selected from the group of: hydrochlorthiazide and chlortaildon. A particularly preferred thiazide diuretic is hydrochlorothiazide.

Inhibitor of the renin-angiotensin system (RAS) include angiotensin converting enzyme (ACE) inhibitors, angiotensin II receptor blockers (ARBs, Angiotensin II receptor antagonists), and direct renin inhibitors. Preferably, the inhibitor is an ACE inhibitor. Also preferably, the inhibitor is an angiotensin II receptor blocker.

Preferably, the ACE-inhibitor is selected from the group consisting of benazepril, captopril, cilazapril, enalapril, fosinopril, lisinopril, moexipril, perindopril, quinapril, ramipril, spirapril, and trandolapril. A particularly preferred ACE-inhibitor is captopril, enalapril, lisinopril, ramipril, or trandolapril.

Preferably, the angiotensin II receptor antagonist is selected from the group consisting of losartan, valsartan, irbesartan, candesartan, olmesartan, telmisartan, and eprosartan. A particularly preferred ARB is valsartan, losartan, candersartan, or telmisartan.

A preferred direct renin inhibitor is aliskiren.

As set forth herein above, the subject to be tested may be treated already with said at least one medicament at the time at which the sample to be tested has been obtained. Thus, by carrying out the method of the present invention, a subject is identified who is eligible to the administration of said at least one medicament at a higher dosage, i.e. at a dosage which is higher than the dosage of said medicament that was administered prior to obtaining the sample to be tested. Preferably, the daily dosage shall be higher. In the context of the present invention it is contemplated to increase the dosage by up to 500% or more. Preferably, said dosage shall be at least 30%, more preferably, at least 50%, even more, preferably, at least 100%, or most preferably, at least 200% higher than the dosage that was administered prior to obtaining the sample to be tested.

The term "subject" as used herein in the context with the aforementioned method relates to animals, preferably mammals, and, more preferably, humans. It is envisaged in the context of the present invention, that the subject suffers from heart failure (HF).

The term "heart failure" as used herein relates to an impaired systolic and/or diastolic function of the heart being accompanied by overt signs of heart failure as known to the person skilled in the art. Preferably, heart failure referred to herein is also chronic heart failure. Heart failure according to the present invention includes overt and/or advanced heart failure. In overt heart failure, the subject shows symptoms of heart failure as known to the person skilled in the art.

HF can be classified into various degrees of severity.

According to the NYHA (New York Heart Association) classification, heart failure patients are classified as belonging to NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;e1-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", in particular, refers to stages B, C and D of the ACC/AHA classification referred to above. In these stages, the subject shows typical symptoms of heart failure. Accordingly, a subject who suffers from heart failure, suffers from heart failure stage B, C or D according to the ACC/AHA classification, in particular stage C or D. Also preferably, the subject may be classified are classified as to belong to NYHA class II; III or IV, in particular class III or IV.

Preferably, the subject in the context of the present invention does not have impaired renal function. Preferably, the subject shall not suffer from renal failure, in particular the subject shall not suffer from acute, chronic and/or end stage renal failure. Further, the subject, preferably, shall not suffer from renal hypertension. How to assess whether a subject exhibits impaired renal function is well known in the art. Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. All calculations derived from formulas such as the Cockgroft Gault formula of the MDRD formula deliver estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of a subject who does not exhibit impaired renal function is within this range. Moreover, said subject preferably, has a blood creatinine level (in particular a serum creatinine level) of lower than 0.9 mg/dl, more preferably of lower than 1.1 mg/dl and most preferably of lower than 1.3 mg/dl.

Preferably, the subject does not suffer from ACS (acute coronary syndrome). The term "ACS" as used herein includes STEMI (ST-elevation myocardial infarction); NSTEMI (non ST-elevation myocardial infarction) and unstable angina pectoris. It is further envisaged that the subject to be tested does not have a history of ACS. In particular, the subject shall not have suffered from ACS within one month prior to carrying out the method of the present invention (to be more precise, within one month prior to obtaining the sample).

As set forth above, the subject to be tested may be treated with the medicaments as set forth in the context of the method of the present invention. Preferably, the subject is treated with a diuretic, beta blocker, an aldosterone antagonist and/or an inhibitor of the renin-angiotensin system. In this case, the method of the present invention allows for identifying a subject being eligible to the administration of a medicament at a higher dosage, or for identified a subject not being eligible to the administration of a medicament at a higher dosage (who preferably may continue the therapy without altering the dosage).

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

As used herein, the term "BNP-type peptides" comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, BNP-type peptides according to the present invention are NT-proBNP, BNP, and variants thereof. BNP is the active hormone and has a shorter half-life than the respective inactive counterpart NT-proBNP. BNP is metabolized in the blood, whereas NT-proBNP circulates in the blood as an intact molecule and as such is eliminated renally. The in-vivo half-life of NT-proBNP is 120 min longer than that of BNP, which is 20 min (Smith 2000, J Endo-crinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measure-ment of the active or the inactive forms of the natriuretic peptide can be advantageous. The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corre-sponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level preferably, at least 50%, 60%, 70%, 80%, 85%, 90%, 92%, 95%, 97%, 98%, or 99% identical to human NT-proBNP, preferably over the entire length of human NT-proBNP. The degree of identity between two amino acid sequences can be determined as described above. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of human NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Lab Invest 230:177-181), Yeo et al. (Yeo 2003, Clinica Chimica Acta 338:107-115).

The term "Growth-Differentiation Factor-15" or "GDF-15" relates to a polypeptide being a member of the transforming growth factor (TGF) cytokine superfamily. The terms polypeptide, peptide and protein are used interchangeable throughout this specification. GDF-15 was originally cloned as macrophage-inhibitory cytokine 1 and later also identified as placental transforming growth factor-15, placental bone morphogenetic protein, non-steroidal anti-inflammatory drug-activated gene 1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF -related cytokines, GDF-15 is synthesized as an inactive precursor protein, which undergoes disulfide-linked homodimerization. Upon proteolytic cleavage of the N terminal pro-peptide, GDF-15 is secreted as a ∼28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific GDF-15 polypeptides, preferably with the amino acid sequence of human GDF-15, more preferably over the entire length of the specific GDF-15, e.g. of human GDF-15. The degree of identity between two amino acid sequences can be determined as described above. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The Insulin like growth factor binding protein (IGFBP) system plays an important role in cell growth and differentiation. It comprises two ligands, IGF-I and IGF-II, two receptors, type 1 and type 2 IGF receptors, and as of 1995 six IGF-binding proteins (IGFBPs), IGFBP-1 to -6 (Jones, J.I., et al., Endocr. Rev. 16 (1995) 3-34). Recently the IGFBP family has been expanded to include the IGFBP-related proteins (IGFBP-rPs), which have significant structural similarities with the IGFBPs (Hwa, V., et al., Endocr. Rev 20 (1999) 761-787). Thus, the IGFBP superfamily includes the six conventional IGFBPs, which have high affinity for IGFs, and at least 10 IGFBP-rPs, which not only share the conserved amino-terminal domain of the IGFBPs but also show some degree of affinity for IGFs and insulin. The IGFBP-rPs are a group of cysteine-rich proteins that control diverse cellular functions, such as cellular growth, cell adhesion and migration, and synthesis of the extracellular matrix. In addition, these proteins might be involved in biological processes like tissue proliferation and differentiation, reproduction, angiogenesis, wound repair, inflammation, fibrosis, and tumorigenesis (Hwa, V., et al., Endocr. Rev 20 (1999)761-787).

IGF binding protein 7 (= IGFBP7) is a 30-kDa modular glycoprotein known to be secreted by endothelial cells, vascular smooth muscle cells, fibroblasts, and epithelial cells (Ono, Y., et al., Biochem Biophys Res Comm 202 (1994) 1490-1496). In the literature this molecule has also been denominated as FSTL2; IBP 7; IGF binding protein related protein I; IGFBP 7; IGFBP 7v; IGFBP rPl; IGFBP7; IGFBPRP1; insulin like growth factor binding protein 7; insulin like growth factor binding protein 7 precursor; MAC25; MAC25 protein; PGI2 stimulating factor; and PSF or Prostacyclin stimulating factor. Northern blot studies revealed a wide expression of this gene in human tissues, including heart, brain, placenta, liver, skeletal muscle, and pancreas (Oh, Y., et al., J. Biol. Chem. 271 (1996) 30322-30325).

IGFBP7 was initially identified as a gene differentially expressed in normal leptomeningeal and mammary epithelial cells, compared with their counterpart tumor cells, and named meningioma-associated cDNA (MAC25) (Burger, A.M., et al., Oncogene 16 (1998) 2459-2467). The expressed protein was independently purified as a tumor derived adhesion factor (later renamed angiomodulin) (Sprenger, C.C., et al., Cancer Res 59 (1999) 2370-2375) and as a prostacyclin stimulating factor (Akaogi, K., et al., Proc Natl Acad Sci USA 93 (1996) 8384-8389). It has additionally been reported as T1Al2, a gene down-regulated in breast carcinomas (StCroix, B., et al., Science 289 (2000) 1197-1202).

Differential expression of IGFBP7 mRNA was measured in patients suffering from various diseases including cardiac disease, kidney disease, inflammatory diseases (US 6,709,855 to Scios Inc.) and vascular graft disease (US 2006/0,003,338).

A number of different assays has been described and used to test for the hormone binding properties of IGFBP7. Low affinity IGF binding was analyzed via competitive affinity cross-linking assays. Recombinant human mac25 protein specifically binds IGF-I and-II (Oh, Y., et al., J. Biol. Chem. 271 (1996) 20322-20325; Kim, H.S., et al., Proc. Natl. Acad. Sci USA 94 (1997) 12981-12986.) IGFBP activity can also be detected by measuring the ability of the protein to bind radiolabeled IGF in Western ligand blotting.

Preferably, the term "IGFBP7" refers to human IGFBP7. The sequence of the protein is well known in the art and is e.g. accessible via GenBank (NP_001240764.1). IGFBP7 as used herein, preferably, encompasses also variants of the specific IGFBP7 polypeptides. For an explanation of the term "variants", please see above.

Immunological determination of circulating IGFBP7 was performed recently. Low levels of this analyte were detected in random human sera and increased serum levels have been seen in association with insulin-resistance (Lopez-Bermejo, A., et al., J. Clinical Endocrinology and Metabolism 88 (2003) 3401-3408, Lopez-Bermejo, A., et al., Diabetes 55 (2006) 2333-2339).

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. It is known that low concentrations of circulating cardiac troponin may be detected in subjects at various conditions, but further studies are required to understand their respective role and rate (Masson et al., Curr Heart Fail Rep (2010) 7:15-21).

The marker Endostatin is well known in the art. Endostatin was originally isolated from murine hemangioendothelioma as a 20 kDA proteolytic fragment of type XVIII collagen (O'Reilly, M.S. et al., Cell 88 (1997) 277-285). Collagens represent a family of extracellular matrix proteins with a characteristic triple-helical conformation forming supra-molecular aggregates that play a dominant role in maintaining tissue structural integrity. Excessive collagen deposition leads to fibrosis disrupting the normal functioning of surrounding tissues. Collagen XVIII is a member of the Multiplexin family of collagens with multiple interruptions in the central triple-helical domain and a unique non-triple-helical domain at the C-terminus mainly in basement membranes. The sequence of the short isoform of human type alpha 1 -chain of collagen XVIII (SwissProt: P39060) is e.g. disclosed in WO2010/124821 which herewith is incorporated by reference with respect to its entire disclosure content.

Endostatin is released from the alpha 1 chain of collagen XVIII by action of various proteolytic enzymes (for details see Ortega, N. and Werb, Z., Journal of Cell Science 115 (2002) 4201-4214 - the full disclosure of this paper is herewith incorporated by reference). Endostatin as used herein is represented by the collagen XVIII fragment spanning from amino acid position 1337 to amino acid position 1519 of collagen XVIII as disclosed in WO2010/124821. The hinge region at the C-terminus of the alpha chain of collagen XVIII contains several protease sensitive sites and a number of enzymes, including neutrophil elastase, cathepsins and matrix metalloproteinases are known to generate endostatin by cleaving the collagen chain in this region. These proteases do not exclusively release endostatin but also may release other, larger fragments that contain the endostatin sequence. As obvious to the skilled artisan such larger fragments will also be measured by an immunoassay for endostatin.

Osteopontin (herein also referred to as "OPN"), also known as bone sialoprotein I (BSP-1 or BNSP), early T-lymphocyte activation (ETA-1), secreted phosphoprotein 1 (SPP1), 2ar and Rickettsia resistance (Ric), is a polypeptide which is a highly negatively charged, extracellular matrix protein that lacks an extensive secondary structure.It is composed of about 300 amino acids (297 in mouse; 314 in human) and is expressed as a 33-kDa nascent protein; there are also functionally important cleavage sites. OPN can go through posttranslational modifications which increase its apparent molecular weight to about 44 kDa. The sequence of ostepontin is well known in the art (human osteopontin: UniProt P10451, GenBank NP_000573.1) Osteopontin is found in normal plasma, urine, milk and bile (US 6,414,219; US 5,695,761; Denhardt, D.T. and Guo, X., FASEB J. 7 (1993) 1475-1482; Oldberg, A., et al., PNAS 83 (1986) 8819-8823; Oldberg, A., et al., J. Biol. Chem. 263 (1988) 19433-19436; Giachelli, CM., et al., Trends Cardiovasc. Med. 5 (1995) 88-95). The human OPN protein and cDNA have been isolated and sequenced (Kiefer M. C, et al., Nucl. Acids Res. 17 (1989) 3306). OPN functions in cell adhesion, chemotaxis, macrophage-directed interleukin-10. OPN is known to interact with a number of integrin receptors. Increased OPN expression has been reported in a number of human cancers, and its cognate receptors (av-b3, av-b5, and av-b1 integrins and CD44) have been identified. In vitro studies by Irby, R.B., et al., Clin. Exp. Metastasis 21 (2004) 515-523 indicate that both endogenous OPN expression (via stable transfection) as well as exogenous OPN (added to culture medium) enhanced the motility and invasive capacity of human colon cancer cells in vitro.

Endostatin is a potent inhibitor of angiogenesis and blood vessel growth. The relationship between endostatin and cytokine networks is undetermined, but it is known that endostatin is able to alter expression of a wide range of genes (Abdollahi, A. et al., MoI. Cell 13 (2004) 649-663).

Endostatin as used herein, preferably, encompasses also variants of the specific endostatin polypeptides. For an explanation of the term "variants", please see above.

Mimecan is a small proteoglycan with leucin-rich repeats and a precursor comprising 298 amino acids. Other names of mimecan are OGN, osteoglycin, OG, OIF, SLRR3A.

Mimecan is a member of the secreted small leucine rich proteoglycans (SLRP) family with structurally related core proteins. The common feature shared by all SLRPs is the tandem leucine-rich repeat (LRR) units in the C-terminal half of the core protein. In the N-terminal region, however, each class of SLRP has a unique domain containing a cysteine cluster with conserved spacing called the LRR N- domain. Class III SLRPs contain six carboxyl LRRs and include mimecan, epiphycan and opticin.

Functional studies from mouse knockouts for class I and II members, such as decorin, biglycan, lumecan and fibromodulin, showed that the SLRP-deficient mice displayed a wide array of defects attributable to abnormal collagen fibrillogenesis suggesting that these SLRPs play important roles in establishing and maintaining the collagen matrix (Ameye, L. and Young, M.F., Glycobiology 12 (2002) 107R-116R). Deficiency of class III mimecan also caused collagen fibril abnormalities (Tasheva, E.S. et al., MoI. Vis. 8 (2002) 407-415).

Mimecan is a multifunctional component of the extracellular matrix. It binds to a variety of other proteins (IGF2, IKBKG, IFNBl, INSR, CHUK, IKBKB, NFKBIA, ILl 5, Cd3, retinoic acid, APP, TNF, lipopolysaccharide, c-abl oncogene 1, receptor tyrosine kinase , v-src sarcoma viral oncogene). These diverse binding activities may account for the ability of mimecan to exert diverse functions in many tissues.

Mimecan has been found in cornea, bone, skin and further tissues. Its expression pattern is altered in different pathological conditions. Despite the increasing amount of data on the biological role of mimecan its function is still not clear. Mimecan has been shown to be involved in regulating collagen fibrillogenesis, a process essential in development, tissue repair, and metastasis (Tasheva et al., MoI. Vis. 8 (2002) 407-415). It plays a role in bone formation in conjunction with TGF-beta-1 or TGF-beta-2.

The sequence of the human mimecan polypeptide is well known in the art and may be assessed, e.g., via GenBank accession number NP_054776.1 GI:7661704. Further, the sequence is disclosed in WO2011/012268. Mimecan as used herein, preferably, encompasses also variants of the specific mimecan polypeptides. For an explanation of the term "variants", please see above. In context of the present invention, mimecan is preferably determined as described in WO2011/012268.

The term "soluble Flt-1" or "sFlt-1" (soluble fins-like tyrosine kinase-1) as used herein refers to polypeptide which is a soluble form of the VEGF receptor Flt1. It was identified in conditioned culture medium of human umbilical vein endothelial cells. The endogenous soluble Flt1 (sFlt1) receptor is chromatographically and immunologically similar to recombinant human sFlt1 and binds [125I] VEGF with a comparable high affinity. Human sFlt1 is shown to form a VEGF-stabilized complex with the extracellular domain of KDR/Flk-1 in vitro. Preferably, sFlt1 refers to human sFlt1. More preferably, human sFlt1 can be deduced from the amino acid sequence of Flt-1 as shown in Genbank accession number P17948, GI: 125361. An amino acid sequence for mouse sFlt1 is shown in Genbank accession number BAA24499.1, GI: 2809071.

The term "sFlt-1" used herein also encompasses variants of the aforementioned specific sFlt-1 polypeptide. Such variants have at least the same essential biological and immunological properties as the specific sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptide. For a more detailed explanation of the term "variants", please see above.

The term "P1GF" (Placental Growth Factor) as used herein refers to a placenta derived growth factor which is a 149-amino-acid-long polypeptide and is highly homologous (53% identity) to the platelet-derived growth factor-like region of human vascular endothelial growth factor (VEGF). Like VEGF, PlGF has angiogenic activity in vitro and in vivo. For example, biochemical and functional characterization of PlGF derived from transfected COS-1 cells revealed that it is a glycosylated dimeric secreted protein able to stimulate endothelial cell growth in vitro (Maqlione1993, Oncogene 8(4):925-31). Preferably, PlGF refers to human PlGF, more preferably, to human PlGF having an amino acid sequence as shown in Genebank accession number P49763, GI: 17380553 (Genebank is available from the NCBI, USA under www.ncbi.nlm.nih.gov/entrez).

In the context of the method of the present invention it is, in particular, envisaged that the amounts of human peptides or polypeptides are determined.

Uric acid is the final product of purine metabolism in a subject organism. The IUPAC name is 7,9-dihydro-3H-purine-2,6,8-trione. The compound is frequently also referred to as urate, Lithic acid, 2,6,8-trioxypurine, 2,6,8-trihydroxypurine, 2,6,8-Trioxopurine, 1H-Purine-2,6,8-triol (compound formual C₅H₄N₄O₃, PubChem CID 1175, CAS number 69-93-2).

Uric acid measurements are used in the diagnosis and treatment of numerous renal and metabolic disorders, including renal failure, gout, leukemia, psoriasis, starvation or other wasting conditions, and of patients receiving cytotoxic drugs. The oxidation of uric acid provides the basis for two approaches to the quantitative determination of this purine metabolite. One approach is the reduction of phosphotungstic acid in an alkaline solution to tungsten blue, which is measured photometrically. A second approach, described by Praetorius and Poulson, utilizes the enzyme uricase to oxidize uric acid; this method eliminates the interferences intrinsic to chemical oxidation (Praetorius E, Poulsen H. Enzymatic Determination of Uric Acid with Detailed Directions. Scandinav J Clin Lab Investigation 1953;3:273-280). Uricase can be employed in methods that involve the UV measurement of the consumption of uric acid or in combination with other enzymes to provide a colorimetric assay. Another method is the colorimetric method developed by Town et al. (Town MH, Gehm S, Hammer B, Ziegenhorn J. J Clin Chem Clin Biochem 1985;23:591) The sample is initially incubated with a reagent mixture containing ascorbate oxidase and a clearing system. In this test system it is important that any ascorbic acid present in the sample is eliminated in the preliminary reaction; this precludes any ascorbic acid interference with the subsequent POD indicator reaction. Upon addition of the starter reagent, oxidation of uric acid by uricase begins.

In the context of the present invention, uric acid can be determined by any method deemed appropriate. Preferably, the biomarker is determined by the aforementioned methods. More preferably, uric acid is determined by applying a slight modification of the colorimetric method described above. In this reaction, the peroxide reacts in the presence of peroxidase (POD), N-ethyl-N-(2-hydroxy-3-sulfopropyl)-3-methylaniline (TOOS), and 4-aminophenazone to form a quinone-diimine dye. The intensity of the red color formed is proportional to the uric acid concentration and is determined photometrically.

Galectin-3 (Gal-3) is a structurally unique member of a family of beta-galactoside-binding lectins. Expression of galectin-3 has been associated with the epithelium and inflammatory cells including macrophages, neutrophils and mast cells. Galectin-3 has been implicated in a variety of biological processes important in heart failure including myofibroblast proliferation, fibrogenesis, tissue repair, cardiac remodeling and inflammation. Galectin-3 is approximately 30 kDa and, like all galectins, contains a carbohydrate-recognition-binding domain (CRD) of about 130 amino acids that enable the specific binding of β-galactosides. Galectin-3 is encoded by a single gene, LGALS3. It comprises an N-terminal domain with tandem repeats of short amino acid segments (a total of 110-130 amino acids) linked to a single C-terminal CRD of about 130 amino acids. It is expressed in the nucleus, cytoplasm, mitochondrion, cell surface, and extracellular space- This protein has been shown to be involved in the following biological processes: cell adhesion, cell activation and chemoattraction, cell growth and differentiation, cell cycle, and apoptosis. Elevated levels of galectin-3 have been found to be significantly associated with higher risk of death in both acute decompensated heart failure and chronic heart failure populations (see, e.g., DeFilippi C, Christenson R, Shah R, et al. (2009). Clinical validation of a novel assay for galectin-3 for risk assessment in acutely destabilized heart failure.)

The protein sequence of Galectin-3 is well known in the art, see e.g. uniprot accession number P17931 (version 5, November 25, 2008), GenBank accession number NP_002297.2 NM_002306.3.

ST2 is a member of the IL-1 receptor family that is produced by cardiac fibroblasts and cardio-myocytes under conditions of mechanical stress. ST2 is an interleukin-1 receptor family member and exists in both membrane-bound isoform and a soluble isoform (sST2). In the context of the present invention, the amount of soluble ST2 shall be determined (see Dieplinger et al. (Clinical Biochemistry, 43, 2010: 1169 to 1170). ST2 also known as Interleukin 1 receptor-like 1 or IL1RL1, is encoded in humans by the IL1RL1 gene. The sequence of the human ST2 polypeptide is well known in the art, and e.g. acessessible via GenBank, see NP_003847.2 GI:27894328. Soluble ST2 (sST2) is believed to function as a decoy receptor by binding IL-33 and abrogating the otherwise cardioprotective effect of IL-33 signaling through the cell membrane-bound form of ST2.

The marker Cystatin C is well known in the art. Cystatin C is encoded by the CST3 gene and is produced by all nucleated cells at a constant rate and the production rate in humans is remarkably constant over the entire lifetime. Elimination from the circulation is almost entirely via glomerular filtration. For this reason the serum concentration of cystatin C is independent from muscle mass and gender in the age range 1 to 50 years. Therefore cystatin C in plasma and serum has been proposed as a more sensitive marker for GFR. The sequence of the human Cystatin C polypeptide can be assessed via Genbank (see e.g. accession number NP_000090.1). The biomarker can be determined by particle enhanced immunoturbidimetric assay. Human cystatin C agglutinates with latex particles coated with anti-cystatin C antibodies. The aggregate is determined turbidimetrically.

The marker Prealbumin is well known by the skilled person. It is a tryptophan-rich protein which is synthesized in hepatocytes and has a molar mass of 55000 daltons. At a pH of 8.6, an electrophoretic band appears prior to albumin in a relative amount of < 2.5 % due to its greater rate of diffusion to the anode. Its function is to bind and transport low molecular weight retinol-binding proteins (molar mass of less than 21000 daltons), preventing their glomerular filtration. 30-50 % of circulating prealbumin is complexed by retinol-binding protein. Furthermore, it binds and transports thyroxine (T4), nevertheless its affinity to this hormone is less than that of thyroxine-binding globulin. The sequence of the human Prealbumin polypeptide can be assessed via Genbank (see e.g. accession number NP_000362.1). Various methods are available for the determination of prealbumin, such as radial immunodiffusion (RID), nephelometry and turbidimetry.

Transferrin (frequently also referred to as Serotransferrin or Beta-1 metal-binding globulin) is a glycoprotein with a molecular weight of about 79570 daltons. It consists of a polypeptide strand with two N-glycosidically linked oligosaccharide chains. Transferrins are iron binding transport proteins which can bind two Fe³⁺ ions in association with the binding of an anion, usually bicarbonate. A variety of methods are available for determining transferrin including radial immunodiffusion, nephelometry and turbidimetry. The sequence of transferrin is well known in the art, see e.g. Schaeffer et al. Gene 56:109-116(1987), or Uniprot accession number P02787, in particular version 178).

P1NP (procollagen type 1 N-terminal propeptide) is a marker for bone formation. It is a specific indicator of type 1 collagen deposition. It is released as a trimeric structure, but degrades to a monomer. Preferably, the total amount of P1NP is measured (total procollagen type 1 N-terminal propeptide).

Determining the amount of a peptide or polypeptide referred to in this specification, in particular of GDF-15 (Growth Differentiation Factor 15), endostatin, mimecan, IGFBP7 (IGF binding protein 7), a cardiac Troponin, a BNP-type peptide, Gal3 (Galectin-3), sST2 (soluble ST2), sFlt-1, PlGF, P1NP, Cystatin C, or prealbumin, relates to measuring the amount or concentration, preferably, semi-quantitatively or quantitatively. Measuring can be done directly or indirectly.

How to determine the amount of uric acid is described herein above. If the biomarker is a peptide or a polypeptide such as GDF-15 (Growth Differentiation Factor 15), endostatin, mimecan, IGFBP7 (IGF binding protein 7), a cardiac Troponin, a BNP-type peptide, uric acid, Gal3 (Galectin-3), or sST2 (soluble ST2), sFlt-1, P1GF, P1NP, Cystatin C, or prealbumin the following applies:
Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys™ analyzers), CBA (an enzymatic Cobalt Bind-ing Assay, available for example on Roche-Hitachi™ analyzers), and latex agglutination assays (available for example on Roche-Hitachi™ analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand.

According to a preferred embodiment, said steps of contacting, removing and measuring may be performed by an analyzer unit of the system disclosed herein. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound ligand, in particular the ligand or the ligand/peptide complex, will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Further suitable techniques for the determination of a polypep-tide or peptide are described in the following.

First, binding of a ligand may be measured directly, e.g. by NMR or surface plasmon resonance. Measurement of the binding of a ligand, according to preferred embodiments, is performed by an analyzer unit of a system disclosed herein. Thereafter, an amount of the measured binding may be calculated by a computing device of a system disclosed herein. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluo-rescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

Preferably, the amounts of the individual biomarkers as referred to herein as determined as described in the Examples section.

The term "amount" as used herein encompasses the absolute amount of a biomarker, the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations. According to preferred embodiments of the subject invention, the determination of an "amount" is performed by the disclosed system, whereby a computing device determines the "amount" based on contacting and measuring steps performed by one or more analyzer units of said system.

The term "comparing" as used herein encompasses comparing the amount of the biomarker, in particular the peptide or polypeptide, comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. Thus, the comparison referred to in step (b) of the method of the present invention may be carried out by a computing device (e.g., of a system disclosed herein). The value of the amount and the reference can be, e.g., compared to each other and the said comparison can be automatically carried out by a computer program executing an algorithm for the comparison. The computer program carrying out the said evaluation will provide the desired assessment in a suitable output format. For a computer assisted comparison, the value of the determined amount may be compared to values corresponding to suitable references which are stored in a database by a computer program. The computer program may further evaluate the result of the comparison, i.e. automatically provide the desired assessment in a suitable output format. For a computer assisted comparison, the value of the determined amount may be compared to values corresponding to suitable references which are stored in a database by a computer program. The computer program may further evaluate the result of the comparison, i.e. automatically provides the desired assessment in a suitable output format. The said result may, preferably, serve as an aid for identifying a subject being eligible to the administration of the at least one medicament as set forth herein elsewhere.

The term "reference amount" (or reference ratio) as used herein, preferably, refers to an amount (or ratio) which allows for allocation of a subject into either the group of subjects who are eligible to the administration of said at least one medicament as set forth in the context of the method of the present invention, or into the group of subjects who are not eligible to the administration of said at least one medicament. Accordingly, the reference amount (or reference ratio) shall allow for identifying a subject being eligible to the administration of at least one medicament.

Such a reference amount (or ratio) can be a threshold amount which separates these groups from each other. The identification may be provided by the computing device of a system disclosed herein based on said comparison of the calculated "amount" (or ratio) to a reference or a threshold. For example, a computing device of a system may provide an indicator, in the form of a word, symbol, or numerical value which is indicative of the identification of the subject.

The reference amount (or reference ratio) applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts (or ratios) can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/- specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the identification of a subject who is eligible to the administration as referred to herein with a proper balance of sensitivity and specificity, respectively. Accordingly, the reference to be used for the aforementioned method of the present invention, i.e. a threshold which allows to discriminate between subjects who are eligible to the administration as referred to herein or those who are not eligible to said administration can be generated, preferably, by establishing a ROC for said cohort as described above and deriving a threshold amount therefrom. Dependent on a desired sensitivity and specificity for a diagnostic method, the ROC plot allows deriving suitable thresholds.

The diagnostic algorithms, i.e. whether administration of a medicament shall be initiated, or whether the dosage of a medicament shall be increased ("uptritrated"), or not, are disclosed in the Examples section. In the following preferred diagnostic algorithms are summarized:
If the at least one medicament is a beta blocker and if the at least one biomarker is endostatin, mimecan, GDF-15, a cardiac Troponin and/or a BNP-type peptide, preferably, the following applies:
   Preferably, an amount (or amounts) of the biomarker (or biomarkers) in the test sample which is (or are) increased as compared to the reference amount (or to the reference amounts) is (or are) indicative for a subject who is eligible to the administration of said at least one medicament, and/or an amount (or amounts) of the biomarker (or the biomarkers) which is (or are) decreased as compared to the reference amount (or to the reference amounts) is (or are) indicative for a subject who is not eligible to the administration of said at least one medicament.

More preferably, the subject to be tested is treated already with a beta blocker. In this case, an amount (or amounts) of the biomarker (or biomarkers) which is (or are) increased as compared to the reference amount (or to the reference amounts) is (or are) indicative for a subject who is eligible to the administration of said medicament at a higher dosage, and/or an amount (or amounts) of the biomarker (or the biomarkers) which is (or are) decreased as compared to the reference amount (or to the reference amounts) is (or are) indicative for a subject who is not eligible to the administration of said medicament at a higher dosage.

If the medicament is a beta blocker and if the biomarker is IGFBP7, P1NP, sFlt-1 or osteopontin, preferably, the following applies:
Preferably, an amount (or amounts) of the biomarker(s) in the test sample which is (or are) decreased as compared to the reference amount(s) is (or are) indicative for a subject who is eligible to the administration of said beta blocker, and/or an amount (or amounts) of the biomarker(s) which is (or are) increased as compared to the reference amount(s) is (or are) indicative for a subject who is not eligible to the administration of said beta blocker. In particular, an amount (or amounts) of the biomarker(s) in the test sample which is (or are) decreased as compared to the reference amount(s) is (or are) indicative for a subject who is eligible to the administration of said beta blocker.

More preferably, the subject to be tested is treated already with a beta blocker. In this case, an amount (or amounts) of the biomarker(s) in the test sample which is (are) decreased as compared to the reference amount(s) is (are) indicative for a subject who is eligible to the administration of said medicament at a higher dosage, and/or an amount (or amounts) of the biomarker(s) which is (or are) increased as compared to the reference amount(s) is indicative for a subject who is not eligible to the administration of said medicament at a higher dosage. In particular, an amount (or amounts) of the biomarker(s) in the test sample which is (or are) decreased as compared to the reference amount(s) is (or are) indicative for a subject who is eligible to the administration of said beta blocker at a higher dosage.

If the at least one medicament is an aldosterone antagonist and if the at least one biomarker is IGFBP7, a cardiac Troponin, Gal-3, Cystatin C, PlGF, GDF-15 and/or sST2, preferably, the following applies:
Preferably, an amount (or amounts) of the biomarker (or biomarkers) in the test sample which is (or are) increased as compared to the reference amount (or to the reference amounts) is (or are) indicative for a subject who is eligible to the administration of said at least one medicament, and/or an amount (or amounts) of the biomarker (or the biomarkers) which is (or are) decreased as compared to the reference amount (or to the reference amounts) is (or are) indicative for a subject who is not eligible to the administration of said at least one medicament.

More preferably, the subject to be tested is treated already with an aldosterone antagonist. In this case, an amount (or amounts) of the biomarker (or biomarkers) which is (or are) increased as compared to the reference amount (or to the reference amounts) is (or are) indicative for a subject who is eligible to the administration of said medicament(s) at a higher dosage, and/or an amount (or amounts) of the biomarker (or the biomarkers) which is (or are) decreased as compared to the reference amount (or to the reference amounts) is (or are) indicative for a subject who is not eligible to the administration of said medicament(s) at a higher dosage.

If the ratio of the amount of PlGF to the amount of sFlt-1 is determined, the following applies as diagnostic algorithm.

Preferably, a ratio of amount of PlGF to the amount of sFlt-1 in the test sample which is increased as compared to the reference ratio is indicative for a subject who is eligible to the administration of said aldosterone antagonist, and/or a ratio which is decreased as compared to the reference ratio is indicative for a subject who is not eligible to the administration of said aldosterone antagonist.

More preferably, the subject to be tested is treated already with an aldosterone antagonist. In this case, a ratio of amount of PlGF to the amount of sFlt-1 in the test sample which is increased as compared to the reference ratio is indicative for a subject who is eligible to the administration of said aldosterone antagonist at a higher dosage, and/or a ratio which is decreased as compared to the reference ratio is indicative for a subject who is not eligible to the administration of said aldosterone antagonist at a higher dosage.

If the at least one medicament is an aldosterone antagonist and if the at least one biomarker is endostatin, uric acid and/or sFlt-1, preferably, the following applies:
Preferably, an amount (or amounts) of the biomarker (or biomarkers) in the test sample which is (or are) decreased as compared to the reference amount (or to the reference amounts) is (or are) indicative for a subject who is eligible to the administration of said at least one medicament, and/or an amount (or amounts) of the biomarker (or the biomarkers) which is (or are) increased as compared to the reference amount (or to the reference amounts) is (or are) indicative for a subject who is not eligible to the administration of said at least one medicament.

More preferably, the subject to be tested is treated already with an aldosterone antagonist. In this case, an amount (or amounts) of the biomarker (or biomarkers) which is (or are) decreased as compared to the reference amount (or to the reference amounts) is (or are) indicative for a subject who is eligible to the administration of said medicament at a higher dosage, and/or an amount (or amounts) of the biomarker (or the biomarkers) which is (or are) increased as compared to the reference amount (or to the reference amounts) is (or are) indicative for a subject who is not eligible to the administration of said medicament at a higher dosage.

If the at least one medicament is an inhibitor of the renin-angiotensin system and if the at least one biomarker is GDF-15, a cardiac Troponin, uric acid, a BNP-type peptide and/or osteopontin, preferably, the following applies:
Preferably, an amount (or amounts) of the biomarker (or biomarkers) in the test sample which is (or are) increased as compared to the reference amount (or to the reference amounts) is (or are) indicative for a subject who is eligible to the administration of said at least one medicament, and/or an amount (or amounts) of the biomarker (or the biomarkers) which is (or are) decreased as compared to the reference amount (or to the reference amounts) is (or are) indicative for a subject who is not eligible to the administration of said at least one medicament.

More preferably, the subject to be tested is treated already with an inhibitor of the renin-angiotensin system. In this case, an amount (or amounts) of the biomarker (or biomarkers) which is (or are) increased as compared to the reference amount (or to the reference amounts) is (or are) indicative for a subject who is eligible to the administration of said medicament at a higher dosage, and/or an amount (or amounts) of the biomarker (or the biomarkers) which is (or are) decreased as compared to the reference amount (or to the reference amounts) is (or are) indicative for a subject who is not eligible to the administration of said medicament at a higher dosage.

If the at least one medicament is a diuretic and the at least one biomarker is selected from the group consisting of IGFBP-7, endostatin, mimecan, GDF-15, prealbumin, transferrin, a BNP-type peptide, and uric acid, preferably, the following applies:
Preferably, an amount (or amounts) of the biomarker (or biomarkers) in the test sample which is (or are) increased as compared to the reference amount (or to the reference amounts) is (or are) indicative for a subject who is not eligible to the administration of said medicament, and/or an amount (or amounts) of the biomarker (or the biomarkers) which is (or are) decreased as compared to the reference amount (or to the reference amounts) is (or are) indicative for a subject who is eligible to the administration of said medicament.

More preferably, the subject to be tested is treated already with a diuretic. In this case, an amount (or amounts) of the biomarker (or biomarkers) which is (or are) increased as compared to the reference amount (or to the reference amounts) is (or are) indicative for a subject who is not eligible to the administration of said medicament at a higher dosage, and/or an amount (or amounts) of the biomarker (or the biomarkers) which is (or are) decreased as compared to the reference amount (or to the reference amounts) is (or are) indicative for a subject who is not eligible to the administration of said medicament at a higher dosage, and/or an amount (or amounts) of the biomarker (or the biomarkers) which is (or are) decreased as compared to the reference amount (or to the reference amounts) is (or are) indicative for a subject who is eligible to the administration of said medicament at a lower dosage.

The following applies if the at least one medicament is an inhibitor of the renin-angiotensin system, and if the at least one biomarker is sFlt-1 and/ or IGFBP7.

Preferably, an amount of the biomarker in the test sample which is increased as compared to the reference amount is indicative for a subject who is not eligible to the administration of said medicament, and/or an amount of the biomarker which is decreased as compared to the reference amount is indicative for a subject who is eligible to the administration of said medicament. More preferably, the subject to be tested is treated already with an inhibitor of the renin angiotensin system. In this case, an amount of the biomarker which is increased as compared to the reference amount is indicative for a subject who is not eligible to the administration of said medicament at a higher dosage, and/or an amount of the biomarker which is decreased as compared to the reference amount is indicative for a subject who is eligible to the administration of said medicament at a higher dosage.

It is also preferred that the reference amount is derived from a subject or group of subjects known to be eligible to the administration of said at least one medicament, and/or from a subject or group of subjects known not be eligible to the administration of said at least one medicament.

In this case, preferred diagnostic algorithms are as follows:
If the at least one medicament is a beta blocker and if the at least one biomarker is endostatin, mimecan, GDF-15, a cardiac Troponin and/or a BNP-type peptide, preferably, the following applies:
   Preferably, the reference amount is derived from a subject or group of subjects known to be eligible to the administration of said at least one medicament, wherein an amount (or amounts) of the biomarker (or biomarkers) in the test sample which is (or are) essentially the same, or which is (or are) increased as compared to the reference amount (or to the reference amounts) is (or are) indicative for a subject who is eligible to the administration of said at least one medicament, and/or the reference amount is derived from a subject or group of subjects known not to be eligible to the administration of said at least one medicament, wherein an amount (or amounts) of the biomarker (or biomarkers) in the test sample which is (or are) essentially the same, or which is (or are) decreased as compared to the reference amount (or to the reference amounts) is (or are) indicative for a subject who is not eligible to the administration of said at least one medicament.

If the medicament is a beta blocker and if the biomarker is IGFBP7, P1NP, sFlt-1 and/or osteopontin, preferably, the following applies:
Preferably, the reference amount for IGPBP7, P1NP, sFlt-1 and/or osteopontin is derived from a subject or group of subjects known to be eligible to the administration of said beta blocker, wherein an amount of IGFBP7, P1NP, sFlt-1 and/or osteopontin in the test sample which is essentially the same, or which is decreased as compared to the reference amount is indicative for a subject who is eligible to the administration of said beta blocker, and/or the reference amount is derived from a subject or group of subjects known not to be eligible to the administration of said beta blocker, wherein an amount of IGFBP7, P1NP, sFlt-1 and/or osteopontin in the test sample which is essentially the same, or which is increased as compared to the reference amount is indicative for a subject who is not eligible to the administration of said beta blocker.

If the at least one medicament is an aldosterone antagonist and if the at least one biomarker is IGFBP7, Cystatin C, a cardiac Troponin, Gal-3, P1GF, GDF-15 and/or sST2, preferably, the following applies:
Preferably, the reference amount is derived from a subject or group of subjects known to be eligible to the administration of said at least one medicament, wherein an amount (or amounts) of the biomarker (or biomarkers) in the test sample which is (or are) essentially the same, or which is (or are) increased as compared to the reference amount (or to the reference amounts) is (or are) indicative for a subject who is eligible to the administration of said at least one medicament, and/or the reference amount is derived from a subject or group of subjects known not to be eligible to the administration of said at least one medicament, wherein an amount (or amounts) of the biomarker (or biomarkers) in the test sample which is (or are) essentially the same, or which is (or are) decreased as compared to the reference amount (or to the reference amounts) is (or are) indicative for a subject who is not eligible to the administration of said at least one medicament.

If the at least one medicament is an aldosterone antagonist and if the at least one biomarker is endostatin, uric acid and/or sFlt-1, preferably, the following applies:
Preferably, the reference amount is derived from a subject or group of subjects known to be eligible to the administration of said at least one medicament (i.e. the aldosterone antagonist), wherein an amount (or amounts) of the biomarker (or biomarkers) in the test sample which is (or are) essentially the same, or which is (or are) decreased as compared to the reference amount (or to the reference amounts) is (or are) indicative for a subject who is eligible to the administration of said at least one medicament; and/or the reference amount is derived from a subject or group of subjects known not to be eligible to the administration of said at least one medicament, wherein an amount (or amounts) of the biomarker (or biomarkers) in the test sample which is (or are) essentially the same, or which is (or are) increased as compared to the reference amount (or to the reference amounts) is (or are) indicative for a subject who is not eligible to the administration of said at least one medicament.

More preferably, the subject to be tested is treated already with an aldosterone antagonist. In this case, an amount (or amounts) of the biomarker (or biomarkers) which is (or are) increased as compared to the reference amount (or to the reference amounts) is (or are) indicative for a subject who is not eligible to the administration of said medicament at a higher dosage, and/or an amount (or amounts) of the biomarker (or the biomarkers) which is (or are) decreased as compared to the reference amount (or to the reference amounts) is (or are) indicative for a subject who is not eligible to the administration of said medicament at a higher dosage.

If the at least one medicament is an inhibitor of the renin-angiotensin system and if the at least one biomarker is GDF-15, a cardiac Troponin, uric acid, a BNP-type peptide and/or osteopontin, preferably, the following applies:
Preferably, the reference amount(s) for the biomarker(s) are derived from a subject or group of subjects known to be eligible to the administration of said inhibitor of the renin-angiotensin system, wherein an amounts of both biomarkers in the test sample which is are essentially the same, and/or which are increased as compared to the reference amounts are indicative for a subject who is eligible to the administration of said inhibitor of the renin-angiotensin system, and/or the reference amount is derived from a subject or group of subjects known not to be eligible to the administration of said inhibitor of the renin-angiotensin system, wherein i) an amount of the biomarker cardiac Troponin in the test sample which is essentially the same, or which is decreased as compared to the reference amount for the cardiac Troponin, or ii) wherein an amounts of both biomarkers in the test sample which is are essentially the same, and/or which are decreased as compared to the reference amounts is indicative for a subject who is not eligible to the administration of said inhibitor of the renin-angiotensin system.

If the at least one medicament is a diuretic and the at least one biomarker is selected from the group consisting of endostatin, mimecan, GDF-15, prealbumin, transferrin, a BNP-type peptide, and uric acid, preferably, the following applies:
Preferably, the reference amount is derived from a subject or group of subjects known to be eligible to the administration of said at least one medicament (i.e. the diuretic), wherein an amount (or amounts) of the biomarker (or biomarkers) in the test sample which is (or are) essentially the same, or which is (or are) decreased as compared to the reference amount (or to the reference amounts) is (or are) indicative for a subject who is eligible to the administration of said at least one medicament, and/or the reference amount is derived from a subject or group of subjects known not to be eligible to the administration of said at least one medicament, wherein an amount (or amounts) of the biomarker (or biomarkers) in the test sample which is (or are) essentially the same, or which is (or are) increased as compared to the reference amount (or to the reference amounts) is (or are) indicative for a subject who is not eligible to the administration of said at least one medicament.

Also preferably, the reference amount is derived from a subject or group of subjects known not to be eligible to the administration of said at least one medicament, wherein an amount (or amounts) of the biomarker (or biomarkers) in the test sample which is (or are) essentially the same, or which is (or are) increased as compared to the reference amount (or to the reference amounts) is (or are) indicative for a subject who is eligible to the administration of said medicament at a lower dosage.

More preferably, the subject to be tested is treated already with a diuretic. In this case, an amount (or amounts) of the biomarker (or biomarkers) which is (or are) increased as compared to the reference amount (or to the reference amounts) is (or are) indicative for a subject who is not eligible to the administration of said medicament at a higher dosage, and/or an amount (or amounts) of the biomarker (or the biomarkers) which is (or are) decreased as compared to the reference amount (or to the reference amounts) is (or are) indicative for a subject who is not eligible to the administration of said medicament at a higher dosage.

The following applies if the at least one medicament is an inhibitor of the renin-angiotensin system, and if the at least one biomarker is sFlt-1 and/or IGFBP7.

Preferably, the reference amount is derived from a subject or group of subjects known to be eligible to the administration of said inhibitor of the renin-angiotensin system, wherein an amount of sFlt-1, and/or IGFBP7 in the test sample which is essentially the same, or which is decreased as compared to the reference amount is indicative for a subject who is eligible to the administration of said inhibitor of the renin-angiotensin system, and/or the reference amount is derived from a subject or group of subjects known not to be eligible to the administration of said inhibitor of the renin-angiotensin system, wherein an amount of sFlt-1, and/or IGFBP7, in the test sample which is essentially the same, or which is increased as compared to the reference amount is indicative for a subject who is not eligible to the administration of said inhibitor of the renin-angiotensin system.

Preferred reference amounts to be applied in the context of the present invention are those described in the Examples. Further preferred reference amounts are as follows:
- GDF-15: within a range between about 3000 and about 5000 ng/ml, in particular about 4000 ng/ml
- endostatin: within a range between about 230 and about 270 ng/ml, in particular about 250 ng/ml
- mimecan: within a range between about 30 and about 70 ng/ml, in particular about 50 ng/ml
- IGFBP7: within a range between about 70 and about 130 ng/ml, in particular about 100 ng/ml
- NT-proBNP: within a range between about 2500 and about 3500 pg/ml, in particular about 3000 pg/ml
- Troponin: within a range between about 22 and about 30 ng/ml, in particular about 26 ng/ml
- uric acid: within a range between about 5 and about 10 ng/ml, in particular about 7.3 ng/ml
- Gal3 (Galectin-3): within a range between about 26 and about 38 ng/ml, in particular about 31.6 ng/ml
- osteopontin: within a range between about 80 and about 120 ng/ml, in particular about 100 ng/ml
- sST2 (soluble ST2): within a range between about 30 and about 38 ng/ml, in particular about 34.0 ng/ml
- sFLT-1: within a range between about 85 and about 111 ng/ml, in particular about 98 ng/ml
- PlGF: within a range between about 15 and about 31 ng/ml, in particular about 20.7 ng/ml
- P1NP: within a range between about 29.0 ng/ml and about 43.5 ng/ml, in particular about 36.72 ng/ml
- Cystatin C: within a range between about 1.20 mg/l and about 2.3mg/l, in particular about 1.76 mg/l
- Prealbumin: within a range between about 0.11 g/l and about 0.27 g/l, in particular about 0.19 g/l
- Transferrin: within a range between about 2.15 g/l and about 2.80 g/l, in particular about 2.48 g/l

If the ratio of PlGF to sFlt-1 is calculated the reference ratio is in particular in the range between about 0.17 and about 0.29. In an embodiment, the reference ratio is 0.23.

Preferably, the aforementioned reference amounts (ratios) are threshold amounts (ratios) which separates these groups referred to herein from each other.

If in the context of the present invention more than one biomarker is determined for assessing whether a subject is susceptible to the administration of a medicament, it is in particular envisaged to compare the determined amounts of the individual biomarkers to reference amounts for the individual biomarkers. E.g. the amount of the biomarker IGFBP7 shall be compared to a reference amount for IGFB7, the amount of the biomarker mimecan to a reference amount for mimecan, the amount of the biomarker endostatin to a reference amount of endostatin, the amount of the biomarker sFlt-1 to a reference amount of sFlt-1 etc. In this case the diagnostic. Moreover, if more than one biomarker is determined, the diagnostic algorithms for the individual biomarkers as set forth herein are preferably combined.

Moreover, the present invention relates to a method of treating a subject with at least one medicament for the therapy of heart failure, comprising
a) determining the amount of at least one biomarker as set forth above in connection with the method of the present invention in a sample from a subject suffering from heart failure, and
b) comparing the amount (or amounts) of the at least one biomarker as determined in step a) with a reference amount (or with reference amounts), whereby a subject being eligible to the administration of said at least one medicament is identified,
c) identifying a subject as being eligible to the administration of at least one medicament,
d) administering said at least one medicament to said subject.

The medicaments are disclosed above. The administration may be the initiation of administration of said at least one medicament, or the administration of said at least one medicament at a higher dosage.

Step c) is based on the results the results of the comparison step b). Diagnostic algorithms are disclosed herein elsewhere.

It was shown in the context of the studies underlying the present invention, that is possible to make decisions with respect to the administration of several medicament classes by using a single biomarker. Therefore, it is envisaged by the method of the present invention to assess whether a subject is eligible to the administration of more than one medicament, in particular by using a single marker.

Thus, in a preferred embodiment of the method of the present invention a subject is identified who eligible to the administration of more than one medicament, in particular to the administration of two or three medicaments. Preferred combinations are as follows:
If the biomarker is GDF-5, the medicaments are preferably:
   i. a beta blocker and an inhibitor of the renin-angiotensin system
   ii. a beta blocker and a diuretic,
   iii. an inhibitor of the renin-angiotensin system and a diuretic, or
   iv. a beta blocker, an inhibitor of the renin-angiotensin system, and a diuretic.

If the biomarker is IGFBP7, the medicaments are preferably: a beta blocker and an aldosterone antagonist. Also, the medicaments are preferably: a beta blocker, an aldosterone antagonist and a diuretic. Further, it is envisaged that the medicaments are a beta blocker, an aldosterone antagonist, a diuretic and an inhibitor of the renin-angiotensin system.

If the biomarker is mimecan, the medicaments are preferably: a beta blocker and a diuretic.

If the biomarker is endostatin, the medicaments are preferably:
i. a beta blocker and an aldosterone antagonist
ii. a beta blocker and a diuretic,
iii. an aldosterone antagonist and a diuretic, or
iv. a beta blocker, an aldosterone antagonist, and diuretic.

If the biomarker is a cardiac Troponin, the medicaments are preferably:
i. a beta blocker and an aldosterone antagonist
ii. a beta blocker and an inhibitor of the renin-angiotensin system,
iii. an aldosterone antagonist and an inhibitor of the renin-angiotensin system, or
iv. a beta blocker, an aldosterone antagonist, and an inhibitor of the renin-angiotensin system.

If the biomarker is uric acid, the medicaments are preferably:
i. a diuretic and an aldosterone antagonist
ii. a diuretic and an inhibitor of the renin-angiotensin system,
iii. an aldosterone antagonist and an inhibitor of the renin-angiotensin system, or
iv. a diuretic, an aldosterone antagonist, and an inhibitor of the renin-angiotensin system.

Accordingly, the present invention relates to a method for identifying a subject being eligible to the administration of two or three medicaments, comprising
a) determining the amount of GDF-15 (Growth Differentiation Factor 15) in a sample from a subject suffering from heart failure, and
b) comparing the amount as determined in step a) with a reference amount, whereby a subject being eligible to the administration of said medicaments is identified,
   wherein the two or three medicaments are
   i. a beta blocker and an inhibitor of the renin-angiotensin system
   ii. a beta blocker and a diuretic,
   iii. an inhibitor of the renin-angiotensin system and a diuretic, or
   iv. a beta blocker, an inhibitor of the renin-angiotensin system, and a diuretic.

Further, the present invention relates to a method for identifying a subject being eligible to the administration of two medicaments, comprising
a) determining the amount of IGFBP7 in a sample from a subject suffering from heart failure, and
b) comparing the amount as determined in step a) with a reference amount (or with reference amounts), whereby a subject being eligible to the administration of said medicaments is identified,
wherein the two medicaments are a beta blocker and an aldosterone antagonist.

In addition, the present invention relates to a method for identifying a subject being eligible to the administration of two medicaments, comprising
a) determining the amount of mimecan in a sample from a subject suffering from heart failure, and
b) comparing the amount as determined in step a) with a reference amount (or with reference amounts), whereby a subject being eligible to the administration of said medicaments is identified,
wherein the two medicaments are a beta blocker and a diuretic.

Accordingly, the present invention relates to a method for identifying a subject being eligible to the administration of two or three medicaments, comprising
a) determining the amount of a cardiac Troponin in a sample from a subject suffering from heart failure, and
b) comparing the amount as determined in step a) with a reference amount (or with reference amounts), whereby a subject being eligible to the administration of said medicaments is identified,
   wherein the two or three medicaments are
   i. a beta blocker and an aldosterone antagonist
   ii. a beta blocker and an inhibitor of the renin-angiotensin system,
   iii. an aldosterone antagonist and an inhibitor of the renin-angiotensin system,
      or
   iv. a beta blocker, an aldosterone antagonist, and an inhibitor of the renin-angiotensin system.

Accordingly, the present invention relates to a method for identifying a subject being eligible to the administration of two or three medicaments, comprising
a) determining the amount of a endostatin in a sample from a subject suffering from heart failure, and
b) comparing the amount as determined in step a) with a reference amount, (or with reference amounts), whereby a subject being eligible to the administration of said medicaments is identified,
   wherein the two or three medicaments are
   i. a beta blocker and an aldosterone antagonist
   ii. a beta blocker and a diuretic,
   iii. an aldosterone antagonist and a diuretic, or
   iv. a beta blocker, an aldosterone antagonist, and diuretic.

If a subject shall be identified who is susceptible to the administration of two or more medicaments based on the determination of the amount a single biomarker, the following shall apply with respect to the reference amount:
Preferably, the reference amount set forth in step b) for the various medicaments may be the same for the respective marker. Accordingly, the amount of the biomarker as determined in step a) of the method of the present invention is, preferably, compared to a single reference amount, and, thus, a reference amount for the individual marker which allows for assessing whether the subject is eligible to the administration of said two or three medicaments, or not (i.e. a reference amount which applies for all medicaments. Also preferably, the reference amount for the individual biomarker with respect to the various medicaments may differ, i.e. it may be medicament-specific. Accordingly, the amount of the biomarker as determined in step a) of the method of the present invention is, preferably, compared to two or three reference amounts. Thus, if a subject shall be identified who is eligible to the administration of two medicaments, the amount of the biomarker shall be compared to two reference amounts. Also, if a subject is identified who is eligible to the administration of three medicaments the reference amount of the biomarker to three reference amounts. The individual reference amounts shall be medicament-specific. Accordingly, individual reference amounts for the various medicaments (with respect to a single marker) may be applied. For example, if a subject shall be identified who is susceptible to the administration of a beta blocker and an aldosterone antagonist, the amount of the biomarker determined in step a) shall be compared to i) a reference amount for said biomarker for identifying a subject being eligible to the administration of beta blocker and to ii) a reference amount for said biomarker for identifying a subject being eligible to the administration of an aldosterone antagonist (this applies e.g. if the biomarker is IGFBP7). For example, if a subject shall be identified who is susceptible to the administration of a beta blocker and a diuretic, the amount of the biomarker determined in step a) shall be compared to i) a reference amount for said biomarker for identifying a subject being eligible to the administration of beta blocker and to ii) a reference amount for said biomarker for identifying a subject being eligible to the administration of a diuretic (this applies e.g. if the biomarker is mimecan).

The diagnostic algorithms for individual biomarkers in combination with the individual medicaments have been set forth elsewhere herein. Moreover, preferred reference amounts were described above. The diagnostic algorithms as well as the preferred reference amounts, preferably, also apply if a subject shall be identified who is susceptible to the administration of two or more medicaments based on the determination of the amount a single biomarker. If a subject shall be identified who is eligible to the administration of two or three medicaments, the diagnostic algorithms set forth herein above for the respective biomarker in combination with the medicaments are combined.

For example, with respect to the biomarker IGFBP7, the following applies:
Preferably, an amount of the biomarker in the test sample which is decreased as compared to the reference amount (in particular a reference amount for identifying a subject being eligible to the administration of a beta blocker) is indicative for a subject who is eligible to the administration of said beta blocker, and/or an amount of the biomarker which is increased as compared to said reference amount is indicative for a subject who is not eligible to the administration of said beta blocker, whereas an amount of the biomarker in the test sample which is increased as compared to the reference amount (in particular a reference amount for identifying a subject being eligible to the administration of an aldosterone antagonist) is indicative for a subject who is eligible to the administration of said aldosterone antagonist, and/or an amount of the biomarker which is decreased as compared to said reference amount is indicative for a subject who is not eligible to the administration of said aldosterone antagonist.

Alternatively, the reference amount for identifying a subject being eligible to the administration of a beta blocker with respect to the biomarker is IGPBP7 is derived from a subject or group of subjects known to be eligible to the administration of said beta blocker, wherein an amount of IGFBP7 in the test sample which is essentially the same, or which is decreased as compared to the reference amount is indicative for a subject who is eligible to the administration of said beta blocker, and/or the reference amount for identifying a subject being eligible to the administration of a beta blocker is derived from a subject or group of subjects known not to be eligible to the administration of said beta blocker, wherein an amount of IGFBP7 in the test sample which is essentially the same, or which is increased as compared to the reference amount is indicative for a subject who is not eligible to the administration of said beta blocker, whereas the reference amount for IGFBP7 for identifying a subject being eligible to the administration of an aldosterone antagonist is derived from a subject or group of subjects known to be eligible to the administration of said aldosterone antagonist, wherein an amount of IGFBP7 in the test sample which is essentially the same, or which is increased as compared to the reference amount is indicative for a subject who is eligible to the administration of said aldosterone antagonist, and/or the reference amount for IGFBP7 for identifying a subject being eligible to the administration of an aldosterone antagonist is derived from a subject or group of subjects known not to be eligible to the administration of aldosterone antagonist, wherein an amount of IGFBP7 in the test sample which is essentially the same, or which is decreased as compared to the reference amount is indicative for a subject who is not eligible to the administration of said aldosterone antagonist.

In an aspect of the invention, a method for establishing an aid for identifying a subject being eligible to the administration of at least one medicament selected from the group consisting of a beta blocker, an aldosterone antagonist, a diuretic, and an inhibitor of the renin-angiotensin system, is contemplated, said method comprising:
a) determining the amount of at least one marker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin by (i) bringing the sample into contact with a detection agent (detection agents) with a detection agent (detection agents) that specifically bind(s) to said at least one marker for a time sufficient to allow for the formation of a complex of the said detection agent and the at least one marker from the sample, (ii) measuring the amount of the formed complex, wherein the said amount of the formed complex is proportional to the amount of the at least one marker present in the sample, and (iii) transforming the amount of the formed complex into an amount of the at least one marker reflecting the amount of the at least one marker present in the sample;
b) comparing said amount to a reference; and
c) establishing an aid for identifying a subject being eligible to the administration of said at least one medicament based on the result of the comparison made in step b).

In another aspect of the invention, a system for establishing an aid for identifying a subject being eligible to the administration of at least one medicament selected from the group consisting of a beta blocker, an aldosterone antagonist, a diuretic, and an inhibitor of the renin-angiotensin system, is contemplated, comprising:
a) an analyzer unit configured to bringing the sample into contact with a detection agent (detection agents) that specifically bind(s) to said at least one marker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2) sFlt-1, PlGF, P1NP, Cystatin C, Prealbumin, and Transferrin for a time sufficient to allow for the formation of a complex of the said detection agent and the at least one marker from the sample,
b) an analyzer unit configured to measure the amount of the formed complex, wherein the said amount of the formed complex is proportional to the amount of the at least one marker present in the sample,
c) a computing device having a processor and in operable communication with said analysis units, and
d) a non-transient machine readable media including a plurality of instructions executable by the processor, the instructions, when executed transform the amount of the formed complex into an amount of the at least one marker reflecting the amount of the at least one marker present in the sample, compare said amount to a reference, and establish an aid for identifying a subject being eligible to the administration of said at least one medicament based on the result of said comparison to said reference.

A suitable detection agent may be, in an aspect, an antibody which is specifically binds to the at least one marker, i.e. a detection agent which binds to GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin in a sample of a subject to be investigated by the method of the invention. Another detection agent that can be applied, in an aspect, may be an aptamere which specifically binds to the at least one marker in the sample. In yet an aspect the, sample is removed from the complex formed between the detection agent and the at least one marker prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the at least one marker present in the sample. It will be understood that the specificity and/or sensitivity of the detection agent to be applied defines the degree of proportion of at least one marker comprised in the sample which is capable of being specifically bound. Further details on how the determination can be carried out are also found elsewhere herein. The amount of formed complex shall be transformed into an amount of at least one marker reflecting the amount indeed present in the sample. Such an amount, in an aspect, may be essentially the amount present in the sample or may be, in another aspect, an amount which is a certain proportion thereof due to the relationship between the formed complex and the amount present in the original sample.

In yet an aspect of the aforementioned method, step a) may be carried out by an analyzer unit, in an aspect, an analyzer unit as defined elsewhere herein.

In an aspect of the method of the invention, the amount(s) determined in step a) is (are) compared to a reference. In an aspect, the reference is a reference as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used are reflected properly. In an aspect, the comparison is carried out automatically, e.g., assisted by a computer system or the like.

The aid for identifying a subject being eligible to the administration of said at least one medicament is established based on the comparison carried out in step b) by allocating the subject either into a group of subjects being eligible to the administration or not being eligible to said administration as set forth herein elsewhere. As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. In an aspect of the invention, the aid for identifying a subject being eligible to the administration of said at least one medicament is established automatically, e.g., assisted by a computing device or the like, as described and disclosed herein.

In an aspect of the method of the invention, said method further comprises a step of recommending and/or managing the subject according to the result established in step c) as set forth elsewhere herein in detail, and/or adapting intensiveness of disease monitoring.

In an aspect of the aforementioned method, steps b) and/or c) are carried out by one or more analyzer units as set forth elsewhere herein.

The present invention also relates to the use of i) at least one biomarker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin or ii) and/or of a detection agent which specifically binds to a biomarker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin in a sample of a subject suffering from heart failure for identifying a subject being eligible to the administration of at least one medicament selected from the group consisting of a beta blocker, an aldosterone antagonist, a diuretic, and an inhibitor of the renin-angiotensin system

The present invention also relates to the use of of i) at least one biomarker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin or ii) and/or of a detection agent which specifically binds to a biomarker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin for the manufacture of a pharmaceutical or diagnostic composition for identifying a subject being eligible to the administration of at least one medicament selected from the group consisting of a beta blocker, an aldosterone antagonist, a diuretic, and an inhibitor of the renin-angiotensin system.

Further, present invention relates to at least one biomarker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin or ii) and/or of a detection agent which specifically binds to a biomarker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin for identifying a subject being eligible to the administration of at least one medicament selected from the group consisting of a beta blocker, an aldosterone antagonist, a diuretic, and an inhibitor of the renin-angiotensin system. Said at least one biomarker or said at least one detection agent may be comprised by a kit.

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker. The term "antibody" has been described elsewhere herein.

According to a preferred embodiment of the present invention, a device adapted for carrying out a method of the invention is provided comprising
a) an analyzer unit comprising a detection agent (or agents) which specifically binds to a marker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin, said unit being adapted for determining the amount(s) of the marker(s) in a sample of a subject suffering heart failure, and
b) an analyzer unit for comparing the determined amount(s) with reference amount(s), whereby a subject is identified who is eligible to the administration of at least one medicament selected from the group consisting of a beta blocker, an aldosterone antagonist, a diuretic, and an inhibitor of the renin-angiotensin sys-tem, said unit comprising a database with a reference amount (or amounts) and a computer-implemented algorithm carrying out the comparison.

Preferred reference amounts and algorithms are disclosed elsewhere herein.

A preferred embodiment of the instant disclosure includes a system for identifying a subject being eligible to the administration of at least one medicament selected from the group consisting of a beta blocker, an aldosterone antagonist, a diuretic, and an inhibitor of the renin-angiotensin system. Examples of systems include clinical chemistry analyzers, coagulation chemistry analyzers, immunochemistry analyzers, urine analyzers, nucleic acid analyzers, used to detect the result of chemical or biological reactions or to monitor the progress of chemical or biological reactions. More specifically, exemplary systems of the instant disclosure may include Roche Elecsys™ Systems and Cobas® e Immunoassay Analyzers, Abbott Architect™ and Axsym™ Analyzers, Siemens Centaur™ and Immulite™ Analyzers, and Beckman Coulter UniCel™ and Acess™ Analyzers, or the like.

Embodiments of the system may include one or more analyzer units utilized for practicing the subject disclosure. The analyzer units of the system disclosed herein are in operable communication with the computing device disclosed herein through any of a wired connection, Bluetooth, LANS, or wireless signal, as are known. Additionally, according to the instant disclosure, an analyzer unit may comprise a stand-alone apparatus, or module within a larger instrument, which performs one or both of the detection, e.g. qualitative and/or quantitative evaluation of samples for diagnostic purpose. For example, an analyzer unit may perform or assist with the pipetting, dosing, mixing of samples and/or reagents. An analyzer unit may comprise a reagent holding unit for holding reagents to perform the assays. Reagents may be arranged for example in the form of containers or cassettes containing individual reagents or group of reagents, placed in appropriate receptacles or positions within a storage compartment or conveyor. Detection reagents may also be in immobilized form on a solid support which are contacted with the sample.Further, an analyzer unit may include a process and/or detection component which is optimizable for specific analysis.

According to some embodiments, an analyzer unit may be configured for optical detection of an analyte, for example a marker, with a sample. An exemplary analyzer unit configured for optical detection comprises a device configured for converting electro-magnetic energy into an electrical signal, which includes both single and multi-element or array optical detectors. According to the present disclosure, an optical detector is capable of monitoring an optical electro-magnetic signal and providing an electrical outlet signal or response signal relative to a baseline signal indicative of the presence and/or concentration of an analyte in a sample being located in an optical path. Such devices may also include, for example, photodiodes, including avalanche photodiodes, phototransistors, photoconductive detectors, linear sensor arrays, CCD detectors, CMOS detectors, including CMOS array detectors, photomultipliers, and photomultiplier arrays. According to certain embodiments, an optical detector, such as a photodiode or photomultiplier, may contain additional signal conditioning or processing electronics. For example, an optical detector may include at least one pre-amplifier, electronic filter, or integrated circuit. Suitable pre-preamplifiers include, for example, integrating, transimpedance, and current gain (current mirror) pre-amplifiers.

Additionally, one or more analyzer unit according to the instant disclosure may comprise a light source for emitting light. For example, a light source of an analyzer unit may consist of at least one light emitting element (such as a light emitting diode, an electric powered radiation source such as an incandescent lamp, an electroluminescent lamp, a gas discharge lamp, a high-intensity discharge lamp, a laser) for measuring analyte concentrations with a sample being tested or for enabling an energy transfer (for example, through florescent resonance energy transfer or catalyzing an enzyme).

Further, an analyzer unit of the system may include one or more incubation units (for example, for maintaining a sample or a reagent at a specified temperature or temperature range). In some embodiments, an analyzer unit may include a thermocycler, include a real-time thermocycler, for subjecting a sample to repeated temperature cycles and monitoring a change in the amount of an amplification product with the sample.

Additionally, an analyzer unit of the system disclosed herein may comprise, or be operationally connected to, a reaction vessel or cuvette feeding unit. Exemplary feeding units include liquid processing units, such as a pipetting unit, to deliver samples and/or reagents to the reaction vessels. The pipetting unit may comprise a reusable washable needle, e.g. a steel needle, or disposable pipette tips. The analyzer unit may further comprise one or more mixing units, for example a shaker to shake a cuvette comprising a liquid, or a mixing paddle to mix liquids in a cuvette, or reagent container.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities. According to some embodiments of the instant disclosure, an algorithm for carrying out a comparison between a determined amount of one or more markers disclosed herein, and a suitable reference, is embodied and performed by executing the instructions. The results may be given as output of parametric diagnostic raw data or as absolute or relative amounts. According to various embodiments of the system disclosed herein, a "diagnosis" may be provided by the computing device of a system disclosed herein based on said comparison of the calculated "amount" to a reference or a threshold. For example, a computing device of a system may provide an indicator, in the form of a word, symbol, or numerical value which is indicative of a particular diagnosis.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Finally, the invention pertains to a kit adapted for carrying out a method of the present invention comprising at least one detection agent which specifically binds to a marker selected from the group consisting of GDF-15 (Growth Differentiation Factor 15), endostatin, mimecan, IGFBP7 (IGF binding protein 7), a cardiac Troponin, a BNP-type peptide, uric acid, Gal3 (Galectin-3), osteopontin, sST2 (soluble ST2), sFlt-1, PlGF, P1NP, Cystatin C, Prealbumin, and Transferrin, reference standards as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a predefined amount for the biomarker as referred to herein representing a reference amount.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

### THE INVENTION

The present invention is directed to:
1. A method for identifying a subject being eligible to the administration of a medicament, said medicament being an aldosterone antagonist, comprising:
   a) determining the amount of the biomarkers PlGF and sFlt-1 in a blood, serum or plasma sample from a subject suffering from heart failure, and
   b1) calculating a ratio of the amount of PlGF to the amount of sFlt-1 (or vice versa), and comparing the calculated ratio to a reference ratio, whereby a subject being eligible to the administration of said aldosterone antagonist is identified, or
   b2) comparing the amounts as determined in step a) with reference amounts, whereby a subject being eligible to the administration of said aldosterone antagonist is identified.
2. The method of clause 1, wherein the administration is the initiation of administration of the aldosterone antagonist, or the administration of the aldosterone antagonist at a higher dosage.
3. The method of any one of clauses 1 or 2, wherein the subject is human.
4. The method of any one of clauses 1 to 3, wherein a ratio of the amount of PlGF to the amount of sFlt-1 in the test sample which is increased as compared to the reference ratio is indicative for a subject who is eligible to the administration of said aldosterone antagonist, and/or wherein a ratio which is decreased as compared to the reference ratio is indicative for a subject who is not eligible to the administration of said aldosterone antagonist.
5. The method of any one of clauses 1 to 4, wherein the subject suffers from heart failure stage B, C or D according to the ACC/AHA classification.
6. Use of i) the biomarkers sFlt-1 and PlGF or ii) of a detection agent which specifically binds to PlGF and a detection agent which specifically binds to sFlt-1, in a blood, serum or plasma sample, of a subject suffering from heart failure for identifying a subject being eligible to the administration of an aldosterone antagonist.

The Figures show:

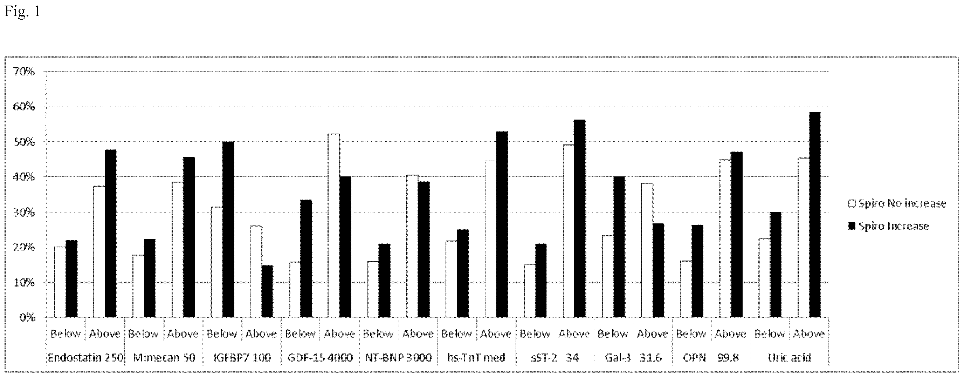

Fig. 1: Effect of adding/uptitrating of an aldosterone antagonist based on marker levels.

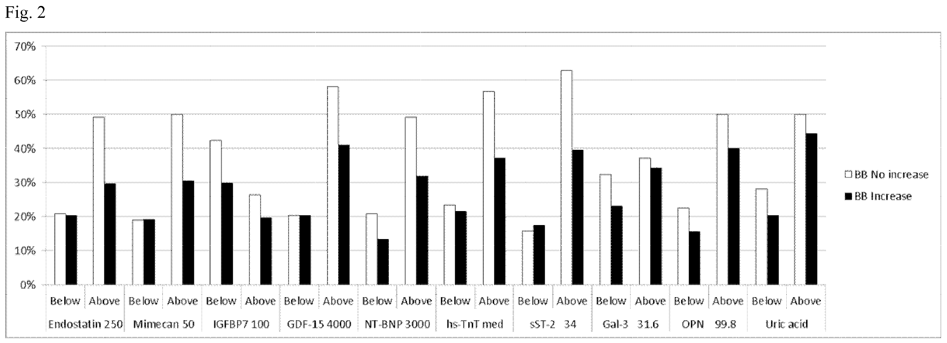

Fig. 2: Effect of adding/uptitrating β-Blocker based on marker levels.

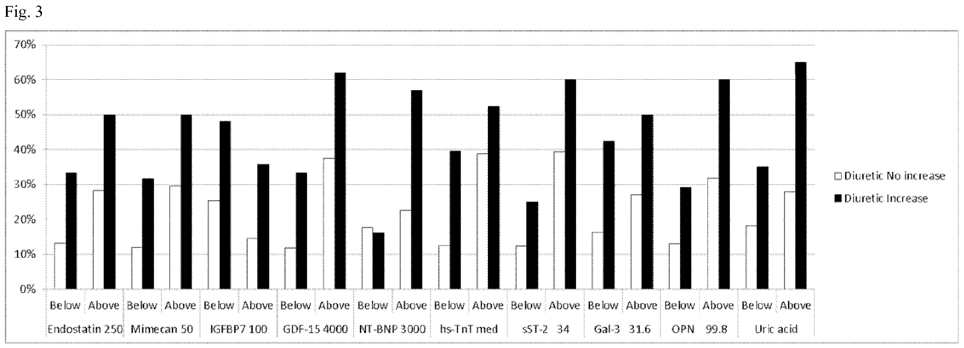

Fig. 3: Effect of adding/uptitrating diuretics based on marker levels.

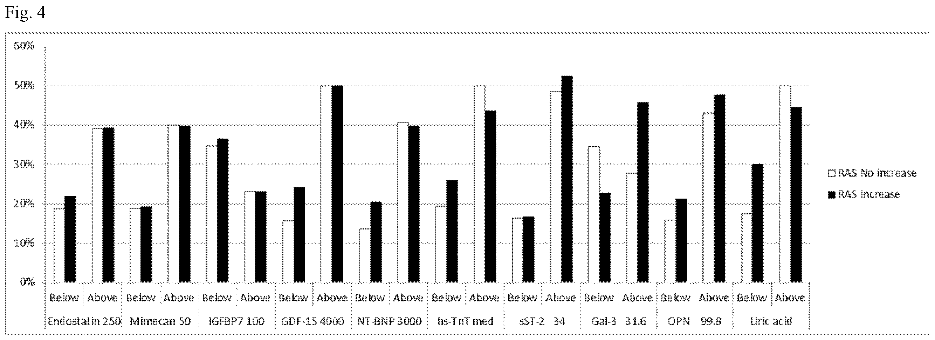

Fig. 4: Effect of adding/uptitrating an inhibitor of the renin-angiotensin system (RAS) based on marker levels.

### EXAMPLES

### (Not all related to the invention)

### Example 1: Assays

The following markers were determined in blood plasma.

Troponin T was determined using Roche's electrochemiluminescence ELISA sandwich test Elecsys Troponin T hs (high sensitive) STAT (Short Turn Around Time) assay. The test employs two monoclonal antibodies specifically directed against human cardiac troponin T. The antibodies recognize two epitopes (amino acid position 125-131 and 136-147) located in the central part of the cardiac troponin T protein, which consists of 288 amino acids. The hs-TnT assay allows a measurement of troponin T levels in the range of 3 to 10000 pg/mL.

NT-proBNP was determined using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

To determine the concentration of GDF-15 in serum and plasma samples, an Elecsys prototype test was employed, using a polyclonal, GDF-15 affinity chromatography-purified, goat anti-human GDF-15 IgG antibody from R&D Systems (AF957). In each experiment, a standard curve was generated with recombinant human GDF-15 from R&D Systems (957-GD/CF). The results with new batches or recombinant GDF-15 protein were tested in standard plasma samples and any deviation above 10% was corrected by introducing an adjustment factor for this assay. GDF-15 measurements in serum and plasma samples from the same patient yielded virtually identical results after correction for eventual dilution factors. The detection limit of the assay was 200 pg/ml.

For detection of IGFBP7 in human serum or plasma, a sandwich ELISA was used. For capture and detection of the antigen, aliquots of an anti-IGFBP7 polyclonal antibody from R&D Systems (Catalogue number: AF 1334) was conjugated with biotin and digoxigenin, respectively.

Streptavidin-coated 96-well microtiter plates were incubated with 100 pi biotinylated anti-IGFBP7 polyclonal antibody for 60 min at 1 pg/ml in lx PBS solution. After incubation, plates were washed three times with lx PBS + 0.02% Tween-20, blocked with PBS + 1% BSA (bovine serum albumen) and then washed again three times with lx PBS + 0.02% Tween-20. Wells were then incubated for 1.5 h with either a serial dilution of the recombinant IGFBP7 as standard antigen or with diluted serum or plasma samples (1:50) from patients or control individuals, respectively. After binding of IGFBP7, plates were washed three times with lx PBS + 0.02% Tween-20. For specific detection of bound IGFBP7, wells were incubated with 100 µl of digoxigenylated anti- IGFBP7 polyclonal antibody for 60 min at 1 µg/ml in lx PBS + 1% BSA. Thereafter, plates were washed three times to remove unbound antibody. In a next step, wells were incubated with 75 mU/ml anti-digoxigenin-POD conjugates (Roche Diagnostics GmbH, Mannheim, Germany, Catalog No. 1633716) for 60 min in lx PBS + 1% BSA. Plates were subsequently washed six times with the same buffer. For detection of antigen-antibody complexes, wells were incubated with 100 µl ABTS solution (Roche Diagnostics GmbH, Mannheim, Germany, Catalog No. 11685767) and the optical density (OD) was measured after 15 min at 405 and 492 nm with an ELISA reader.

Gal-3 was determined by using the BGM Galectin-3 assay (BG medicine, Waltham, MA, USA). It quantitatively measures galectin-3 in serum or EDTA-plasma by enzyme linked immunosorbent assay (ELISA) on a microtiter plate platform. tilizes two monoclonal antibodies against galectin-3. One rat monoclonal anti-mouse galectin-3 antibody is coated onto the surface of the wells in a microtiter plate and serves as the capture antibody to bind galectin-3 molecules in samples, while the other mouse monoclonal anti-human galectin-3 antibody is provided in solution and functions as the tracer antibody for detecting galectin-3 molecules bound to the capture antibody.

For detection of mimecan in human serum or plasma, a sandwich ELISA was used. For capture and detection of the antigen, aliquots of an anti-mimecan polyclonal antibody from R&D Systems (Catalogue number: AF 2660) are conjugated with biotin and digoxygenin, respectively. Streptavidin-coated 96-well microtiter plates are incubated with 100 µl biotinylated anti-mimecan polyclonal antibody for 60 min at 0.2 [mu]g/ml in Ix PBS solution. After incubation, plates are washed three times with 1x PBS + 0.02% Tween-20, blocked with PBS + 2% BSA (bovine serum albumen) for 45 min and then washed again three times with Ix PBS + 0.02% Tween-20. Wells are then incubated for 1 h with 100 µl of either a serial dilution of the recombinant mimecan as standard antigen or with diluted serum or plasma samples (1:5 in 1x PBS + 1%BSA) from patients or control individuals, respectively. After binding of mimecan, plates are washed three times with Ix PBS + 0.02% Tween-20. For specific detection of bound mimecan, wells are incubated with 100µl of digoxigenylated anti-mimecan polyclonal antibody for 45 min at 0.2 µg/ml in Ix PBS + 1% BSA. Thereafter, plates are washed three times to remove unbound antibody. In a next step, wells are incubated with 100 µl of 75 mU/ml anti-digoxigenin-POD conjugates (Roche Diagnostics GmbH, Mannheim, Germany, Catalog No. 1633716) for 30 min in 1x PBS + 1% BSA. Plates are subsequently washed six times with the same washing buffer as above. For detection of antigen-antibody complexes, wells are incubated with 100 µl ABTS solution (Roche Diagnostics GmbH, Mannheim, Germany, Catalog No. 11685767) and the optical density (OD) is measured after 15 min at 405 and 492 nm with an ELISA reader.

For measurement of endostatin in human serum or plasma, a commercially available sandwich ELISA (Quantikine Human Endostatin Immunoassay, Catalog Number DNSTO, R&D Systems) was used. Measurements are performed according to the instructions given by the manufacturer.

sST2 was determined by using the Presage™ ST2 Assay from Critical Diagnostics (San Diego, CA, USA). The assay is a quantitative sandwich monoclonal ELISA in a 96 well plate format for measurement of ST2 in serum or plasma. Diluted plasma was loaded into appropriate wells in the anti-ST2 antibody coated plate and incubated for the prescribed time. Following a series of steps where reagents are washed from the plate, and additional reagents were added and subsequently washed out, the analyte was finally detected by addition of a colorimetric reagent and the resulting signal was measured spectroscopically at 450 nm.

The biomarker mimecan was determined as described in WO2011/012268.

PlGF and sFlt1 were tested using an ELECSYS immunoassay which employs two antibodies that are specific for PlGF and sFlt1, respectively.. The test can be carried out automatically using different Roche analysers including ELECSYS 2010 and cobra e411 and cobra e601. The test has a sensitivity of 3 pg/ml with respect to PlGF. sFlt-1 amounts between 10 to 85,000 pg/ml.

Prealbumin was tested using an in vitro test for the quantitative determination of prealbumin in human serum on Roche/Hitachi cobas c systems (ACN 710 for c 311/501 analyzers; ACN 8710 for c 520 analyzers; Cat. No. 20764655 322). The assay is an immunoturbidimetric assay. Human prealbumin forms a precipitate with a specific antiserum which is determined turbidimetrically.

Cystatin C was used by using an immunoturbidimetric assay for the quantitative in vitro determination of cystatin C in human serum and plasma on Roche automated clinical chemistry analyzers (For Roche/Hitachi 917, MODULAR P analyzers: ACN 431, Cat. No. 04975774 190). Human cystatin C agglutinates with latex particles coated with anti-cystatin C antibodies. The aggregate is determined turbidimetrically at 546 nm.

### Example 2: Patient cohort/Results

Examples from TIME-CHF study: GDF-15, TnT-hs, uric acid, Endostatin, IGFBP-7, Mimecan, sST2, Galectin-3 and osteopontin levels have been determined in samples of n= 450 patients from the TIME-CHF randomized Trial (aged 60 years or older with systolic HF (ejection fraction </= 45%), NYHA class of II or greater prior hospitalization for HF within 1 year and NTproBNP level of 2 or more times the upper limit of normal). The TIME-CHF Study is described in BNP-Guided vs Symptom-Guided Heart Failure Therapy JAMA, 2009; 301 (4):383-392.

At baseline, most patients were receiving recommended HF Therapy ACE Inhibitors or Angiotensin II receptor blockers, β-blocker, diuretics.

The biomarkers were measured at baseline and after 6 month. The numbers in the following table given is the % of patients with "poor outcome" (death, repeated hospitalization,). It was analyzed whether the measured biomarkers would allow for identifying patients which would benefit from increasing the dosage or reducing the dosing interval of beta blocker, an aldosterone antagonist, a diuretic, and/or an inhibitor of the renin-angiotensin system and/or adding the certain medicaments the therapy. For this analysis, the patients were divided into i) a group of subjects which received an increased dosage of the medicaments and/or which received an additional medicament and ii) a group of subjects in which the treatment regimen was not amended. In a further step the two groups were each divided into groups based on the respective biomarker level (below the median/above a threshold). The results are shown in Table 1:

**Table 1: Effect of Therapy Selection based on Marker Level (RAS: Inhibitors of the renin-angiotensin system e.g. ACEi, ARBs, BB: β-blocker, Spiro: spironolactone, aldosterone antagonists)**

| | | RAS | | BB | | Diuretic | | Spiro | |
|---|---|---|---|---|---|---|---|---|---|
| | | No increase | Increase | No increase | Increase | No increase | Increase | No increase | Increase |
| Endostatin 250 | Below | 18,8% | 22,0% | 20,8% | 20,4% | 13,2% | 33,3%*** | 20,0% | 21,9% |
| | Above | 39,2% | 39,3% | 49,1% | 29,6%*** | 28,3% | 50,0%*** | 37,2% | 47,6% |
| Mimecan 50 | Below | 18,9% | 19,2% | 18,9% | 19,2% | 11,9% | 31,6%*** | 17,6% | 22,2% |
| | Above | 40,0% | 39,7% | 50,0% | 30,4%*** | 29,6% | 50,0%*** | 38,4% | 45,5% |
| IGFBP7 100 | Below | 34,8% | 36,5% | 42,3% | 29,8%* | 25,4% | 48,0%*** | 31,3% | 50,0%** |
| | Above | 23,1% | 23,1% | 26,4% | 19,6% | 14,5% | 35,7%*** | 26,0% | 14,8% |
| GDF-15 4000 | Below | 15,7% | 24,2% | 20,3% | 20,4% | 11,8% | 33,3%**** | 15,7% | 33,3% |
| | Above | 50,0% | 50,0% | 58,1% | 41,0%* | 37,5% | 61,9%*** | 52,2% | 40,0% |
| NT-BNP 3000 | Below | 13,6% | 20,4% | 20,8% | 13,3% | 17,7% | 16,1% | 15,9% | 20,8% |
| | Above | 40,7% | 39,7% | 49,2% | 31,8%*** | 22,6% | 56,9%**** | 40,6% | 38,7% |
| hs-TnT med | Below | 19,3% | 25,9% | 23,4% | 21,6% | 12,5% | 39,5%*** | 21,8% | 25,0% |
| | Above | 50,0% | 43,5% | 56,8% | 37,2%** | 38,9% | 52,3% | 44,4% | 52,9% |
| sST-2 34 | Below | 16,3% | 16,7% | 15,7% | 17,4% | 12,3% | 25,0% | 15,1% | 20,8% |
| | Above | 48,4% | 52,4% | 62,9% | 39,5% | 39,4% | 60,0% | 49,1% | 56,2% |
| Gal-3 31.6 | Below | 34,4% | 22,7% | 32,4% | 23,1% | 16,3% | 42,4% | 23,2% | 40,0% |
| | Above | 27,9% | 45,7% | 37,2% | 34,3% | 27,1% | 50,0% | 38,1% | 26,7% |
| OPN 99.8 | Below | 15,8% | 21,3% | 22,5% | 15,6% | 13,0% | 29,0% | 16,1% | 26,1% |
| | Above | 42,9% | 47,6% | 50,0% | 40,0% | 31,8% | 60,0% | 44,8% | 47,1% |
| Uric acid | Below | 17,4% | 30,0%* | 28,1% | 20,4% | 18,2% | 35,0%** | 22,4% | 30,0% |
| | Above | 50,0% | 44,4% | 50,0% | 44,4% | 27,8% | 65,0%*** | 45,3% | 58,3% |

| | | | | | | | | | |
|---|---|---|---|---|---|---|---|---|---|
| Explanation of the presentation of the results in Table 1: For example patients with an endostatin value at baseline of less than 250 receiving an increase in beta-blocker (BB) during the first 6 months have an event-rate of 20.4% as compared to 20.8% without increase. In contrast, the event rates differed significantly (p<0.05) in those with endostatin levels above 250 where patients with an increase in BB had 29.6%, but those without an increase in BB of 49.1%. p-values: * 0.1 - 0.2 ** 0.05 - 0.1 *** 0.01 - 0.05 **** <0.01 | | | | | | | | | |

The data was further analyzed using functional principal component analysis (fPCA). fPCA reduces the dimensionality of the complex dosing data of drugs to allow for interaction analysis with marker levels and outcomes at various time points (beyond baseline). In this case, three principal components were used representing 1) the overall dose level of a drug during the study (most important component), 2) the trend of a drug dose to increase or decrease during the study, and 3) the trend of a drug dose to first increase and then decrease or first decrease and then increase. The advantage and complementary information of the fPCA over the stratification analysis at baseline (previous analysis) is that it reveals interaction of markers and drugs at time points beyond baseline. Therefore, additional interactions and therapy response predictions can be assessed. The components of the fPCA were then tested for interaction and therapy response prediction with median marker levels and outcomes using the same endpoint as described above. The outcome and strength of the interaction is represented in the table below. These results confirm many of the previously found interactions and suggest additional marker-dug-combinations.

The results are shown in the following table:

**Table 2: Functional Principal Component Analysis**

| Drug class Components | Loop | | | Diuretics | | | RAS | | | BB | | | Spiro | | |
|---|---|---|---|---|---|---|---|---|---|---|---|---|---|---|---|
| | *1 | *2 | *3 | *1 | *2 | *3 | *1 | *2 | *3 | *1 | *2 | *3 | *1 | *2 | *3 |
| IGFBP7 | | | | 1 | | | | 3 | | 4 | 1 | 1 | | 1 | 4 |
| Endostatin | | 1 | | | 1 | | | 1 | | | | | 1 | | 2 |
| Mimecan | | | | | | | | 3 | | 3 | 2 | | | 2 | |
| sST2 | | | | | | | | | | | | 3 | 2 | 1 | |
| Gal-3 | | | 3 | 1 | | 2 | | | 3 | | | | 3 | 1 | |
| Hs-cTnT | | 3 | 1 | | 3 | 2 | 1 | | 1 | | | | | | |
| sFlt-1 | | | 2 | | | | 4 | | 3 | | 2 | | 2 | | 3 |
| PLGF | | 1 | | | 1 | | | | 4 | | | 3 | 4 | | |
| P1NP | | | | | | | | | | 3 | 3 | | | | |
| Cystatin C | | | | | | | | | | | | | 4 | | |
| Prealbumin | 3 | | | 2 | | | | | | | | | | | |
| Transferrin | 2 | | | | 2 | | | | | | | | | | |
| Osteopontin | | | 2 | | | | 3 | | 1 | | | | | | 3 |
| GDF-15 | | | | 3 | 1 | | 3 | | | | | | | | 1 |
| Uric acid | 2 | | | 2 | | | 1 | | | | 2 | | 1 | 2 | |
| NT-proBNP | | | 1 | | 1 | | 3 | | 1 | 2 | 3 | | 1 | | |

| | | | | | | | | | | | | | | | |
|---|---|---|---|---|---|---|---|---|---|---|---|---|---|---|---|
| Level of significance of interaction term 1 0.1 - 0.2 2 0.05 - 0.1 3 0.01 - 0.05 4 <0.01 | | | | | | | | | | | | | | | |

Results for IGFBP7: Data evaluation showed that patients with IGFBP-7 levels (independent from other biomarker) below a reference value at baseline (in the study < 100 ng/mL) do not benefit from addition or up-titration of spironolactone. If IGFBP-7 levels are above a reference value (> 100 ng/mL in the study, cut-off to be defined; age dependent, in younger population usually lower levels found), aldosterone antagonists (e.g. spironolactone) and/or beta blockers should be added or up-titrated. 18 months survival in the second group improved while the survival rate does not change in the first group.

Results for GDF-15: Data evaluation showed that patients with GDF-15 levels (independent from other biomarker) below a reference value at baseline (in the study < 4000 pg/mL) do not benefit from additional administration or uptitration of β-blocker or aldosterone antagonists (e.g. spironolactone), while patients with GDF-15 levels above a reference value (> 4000 pg/mL in the study) are likely to benefit from addition or uptitration of β-blocker or aldosterone antagonists. 18 months survival in the second group improved while the survival rate does not change in the first group.

Results for endostatin: Data evaluation showed that patients with endostatin levels (independent from other biomarker) below a reference value at baseline (in the study < 250 ng/mL) do not benefit from additional administration or uptitration of β-blocker. Patients already on a β-blocker compound should remain, but the dose does not need to be increased. Patients with endostatin levels above a reference value (> 250 ng/mL in the study) are likely to benefit from additional administration of β-blocker. Additionally, uptitration of diuretics should be avoided. 18 months survival in the second group improved while the survival rate does not change in the first group.

Results for mimecan: Data evaluation showed that patients with mimecan levels (independent from other biomarker) below a reference value at baseline (in the study < 50 ng/mL) do not benefit additional administration or from uptitration of a β-blocker. If mimecan levels are above a reference value (> 50 ng/mL in the study), a β-blocker should be added or the dose of β-blocker should be uptitrated. Additionally, uptitration of diuretics should be avoided.

Results for NTproBNP: Data evaluation showed that patients with NTproBNP levels above a reference value (> 3000 pg/mL in the study), do not benefit from addition or uptitration of diuretics.

Results for sST2: Data evaluation showed that patients with sST2 levels above a reference value (>34.0 g/mL in the study), benefit from addition or uptitration of a β-blocker.

Results for Gal-3: Data evaluation showed that patients with Gal-3 levels below a reference value (<31.6 g/mL in the study), benefit from addition or uptitration of a inhibitor of the renin-angiotensin system.

Results for sFlt-1: Data evaluation showed that patients with sFlt-1 levels below a reference value (<98 g/mL in the study), benefit from addition or uptitration of a RAS inhibitors and/or aldosterone antagonists.

Results for PlGF: Data evaluation showed that patients with sFlt-1 levels above a reference value (>20.7 g/mL in the study), benefit from addition or uptitration of an aldosterone antagonist.

Results for osteopontin: Data evaluation showed that patients with osteopontin levels above a reference value (>100g/mL in the study), do not benefit from addition or uptitration of diuretic therapy

The following conclusions maybe drawn:
- If Endostatin is above the reference amount, a BB should be added and/or their dose should be uptitrated. Additionally, uptitration of aldosterone antagonists and/or diuretics should be avoided.
- If Mimecan is above the reference amount, a BB should be added and/or the dose of the BB should be uptitrated. Additionally, uptitration of diuretics should be avoided.
- If IGFBP7 is below the reference amount, BB should be added or uptitrated. If IGFBP7 is above the reference amount an aldosterone antagonists should be added or uptitrated
- If IGFBP7 is below the reference amount, aldosterone antagonists and RAS inhibitors should NOT be added or uptitrated
- If GDF-15 is above the reference amount, a BB, RAS inhibitor and aldosterone antagonists should be added or uptitrated. In contrast, a diuretic should not be added or the diuretic dose should not be increased.
- If cTnT-hs is above median, RAS inhibitors, BB compounds or aldosterone antagonists can be uptitrated while this should not be done when cTnT-hs is below the median
- If uric acid is above the median , RAS-inhibitor compounds can be uptitrated. Additionally, uptitration of diuretics and/or aldosterone antagonist should be avoided.
- Patients with NTproBNP levels above a reference amount (in this study > 3000 pg/mL) do not benefit from uptitration of diuretics, but RAS inhibitorsand BB should be uptitrated. Patients with NTproBNP levels below a reference amount (in this study < 3000 pg/mL) do not benefit from uptitration of RAS inhibitors.
- If Gal-3 is above the median, aldosterone antagonists should be added and/or uptitrated.
- If Osteopontin is above the median, RAS inhibitors should be added and/or uptitrated. If Osteopontin is below the median, BB compounds should be added and/or uptitrated.
- If sFlt-1 is below the reference amount (in this studies the median was used), RAS inhibitors, BB compounds, and/or aldosterone antagonists should be added and/or uptitrated
- If PLGF is above the reference amount (e.g. the median), aldosterone antagonists should be added and/or uptitrated
- If sST2 is above the reference amount (e.g. the median), aldosterone antagonists and/or beta blockers should be added and/or uptitrated.
- If P1NP is below the reference amount (e.g. the median), beta blockers should be added and/or uptitrated.
- If Prealbumin is above the reference amount (e.g. the median), diuretics should not be added, the dose decreased or not uptitrated.
- If Transferrin is above the reference amount (e.g. the median), diuretics should not be added, the dose decreased or not uptitrated.
- If Cystatin C is below the reference amount (e.g. the median), aldosterone antagonists should not be added or uptitrated, high doses should be decreased
- If the ratio PlGF/sFlt-1 C is above the reference ratio (e.g. median), aldosterone antagonists should be added or the dose uptitrated.

Thus, by applying the diagnostic algorithms summarized above, a subject can be identified who is eligible to administration (i.e. the initial administration or the administration at a higher dosage, "uptitration") of the medicaments referred to above. Suitable reference amounts can be determined as described in the specification.

### Example 3: Individual case studies

A 89 year old male patient with class C heart failure is receiving low doses of chlortalidon (25 mg/d), enalapril (5 mg/d), and metoprolol (25 mg/d). The patient shows signs of progression of heart failure with elevated NT-proBNP levels. The patient also has asthma and the treating physician is in doubt as to whether the BB should be uptitrated. Mimecan is determined in a plasma sample obtained from the patient. The Mimecan value is above 80 pg/mL. The therapy is intensified by means of sequential uptitration of enalapril (to 20 mg/d) and metoprolol (100 mg/d). In contrast, the chlortalidon dose is not increased. The patient remains stable with a good outcome until the end of the study (no death or hospitalization).

A 90 year old female patient with class C heart failure is receiving a combined a fixed dose combination of hydrochlorothiazide (12.5 mg/d) and valsartan (80 mg/d), as well as atenololol (100 mg/d). The patient has had episode of decompensation and hospitalization in the past. In the last visit, the patient's exercise intolerance worsened and the 6-min walking test yielded a reduced walking distance compared to the last visit 3 months ago. NT-ProBNP and IGFBP-7 are determined in a plasma sample obtained from the patient. The NT-proBNP value is above 1000 pg/mL and the IGFBP-7 value is above 100 pg/ml. The treating physician is not sure whether to safely add spironolactone since the patient has occasionally presented with elevated potassium levels. The therapy is intensified and spironolactone is added starting at 25 mg/d and later on uptitrated to 100 mg/d while the serum porassium level is closely monitored. As a consequence, NT-proBNP value falls below 1000 pg/mL. The patient remains stable with a good outcome until the end of the study (no death, no hospitalization).

A 93 year old male patient with class D heart failure is receiving hydrochlorothiazide (25 mg/d) and valsartan (160 mg/d), as well as bisoprolol (2.5 mg/d). The patient has had episode of decompensation and prolonged hospitalization in the past. During the past visit, NT-ProBNP and IGFBP-7 are determined in a plasma sample obtained from the patient. The NT-proBNP value is above 1000 pg/mL and the IGFBP-7 value is below 100 pg/ml. The treating physician does not add spironolactone. Instead, the therapy is intensified by means of uptitration of bisoprolol to 10 mg/d. The patient remains stable with a good outcome until the end of the study (no death, no hospitalization).

A 70 year old female patient with class B heart failure is receiving captopril (2 x 6.25 mg/d). The patient has been stable for 10 months, but has been hospitalized for acute heart failure after consuming a bag of potato chips watching TV. GDF-15 and cTnThs are determined in a plasma sample obtained from the patient at a pre-scheduled visit. The GDF-15 value is above 4000 pg/mL and cTnT-hs is above the median value. Addition of the beta blocker metoprolol and the aldosterone antagonist spironolactone are selected to intensify the therapy. The patient experienced just one more episode of hospitalization due to dizziness and hypotension.

A 77 year old male vegetarian patient with class C heart failure is receiving valsartan (160 mg/d), as well as carvedilol (12.5 mg/d). The patient has been stable during the past 5 months, but arrives at the emergency department in a decompensated state with acute dyspnea, rales, and tachycardia. Uric acid and Gal-3 are determined in a plasma sample obtained from the patient. The Uric acid and Gal-3 values are below the median. The treating physician intensifies the therapy by means of uptitration of carvedilol to 50 mg/d. Furthermore, Spironolactone is added starting at 25 mg/d and later on uptitrated to 50 mg/d. The patient remains stable for 1 month, but then develops edema, atrial fibrillation, dyspnea, and coughing. On the way to the hospital, the patient dies due to sudden death.

### CONCLUSIONS:

The present disclosure provides a method for the selection of drug therapies that will be beneficial in patients suffering from heart failure. The method predicts responses to therapy and/or help to select an appropriate therapy or therapy intensification. Compared to prior art and to previous biomarker guided heart failure approaches, the method provides specific target levels of various markers and specific indications for therapy selection and dosing. The method also improves the identification and utilization of drug therapies to the benefit of patients and conversely to avoid therapies that are potentially harmful to patients. Thus, the method aims at reducing the mortality and morbidity in heart failure patients.

## Claims

1. A method for identifying a subject being eligible to the administration of a medicament, said medicament being an aldosterone antagonist, comprising
a) determining the amount of the biomarkers PlGF and sFlt-1 in a blood, serum or plasma sample from a subject suffering from heart failure, and
b1) calculating a ratio of the amount of PlGF to the amount of sFlt-1 (or vice versa), and comparing the calculated ratio to a reference ratio, whereby a subject being eligible to the administration of said aldosterone antagonist is identified, or
b2) comparing the amounts as determined in step a) with reference amounts, whereby a subject being eligible to the administration of said aldosterone antagonist is identified.

2. The method of claim 1, wherein the administration is the initiation of administration of the aldosterone antagonist, or the administration of the aldosterone antagonist at a higher dosage.

3. The method of any one of claims 1 or 2, wherein the subject is human.

4. The method of any one of claims 1 to 3, wherein a ratio of the amount of PlGF to the amount of sFlt-1 in the test sample which is increased as compared to the reference ratio is indicative for a subject who is eligible to the administration of said aldosterone antagonist, and/or wherein a ratio which is decreased as compared to the reference ratio is indicative for a subject who is not eligible to the administration of said aldosterone antagonist.

5. The method of any one of claims 1 to 4, wherein the subject suffers from heart failure stage B, C or D according to the ACC/AHA classification.

6. Use of i) the biomarkers sFlt-1 and PlGF or ii) of a detection agent which specifically binds to PlGF and a detection agent which specifically binds to sFlt1, in a blood, serum or plasma sample of a subject suffering from heart failure, for identifying a subject being eligible to the administration of an aldosterone antagonist.

## Patentansprüche

1. Verfahren zum Identifizieren eines für die Verabreichung eines Medikaments geeigneten Subjekts, wobei es sich bei dem Medikament um einen Aldosteronantagonisten handelt, umfassend
a) die Bestimmung der Menge der Biomarker PlGF und sFlt-1 in einer Blut-, Serum- oder Plasmaprobe von einem an Herzinsuffizienz leidenden Subjekt und
b1) die Berechnung eines Verhältnisses der Menge an PlGF zur Menge an sFlt-1 (oder umgekehrt) und das Vergleichen des berechneten Verhältnisses mit einem Vergleichsverhältnis, wodurch ein für die Verabreichung des Aldosteronantagonisten geeignetes Subjekts identifiziert wird, oder
b2) das Vergleichen der in Schritt a) bestimmten Mengen mit Vergleichsmengen, wodurch ein für die Verabreichung des Aldosteronantagonisten geeignetes Subjekt identifiziert wird.

2. Verfahren nach Anspruch 1, wobei es sich bei der Verabreichung um die Einleitung der Verabreichung des Aldosteronantagonisten oder die Verabreichung des Aldosteronantagonisten in einer höheren Dosierung handelt.

3. Verfahren nach Anspruch 1 oder 2, wobei das Subjekt menschlich ist.

4. Verfahren nach einem der Ansprüche 1 bis 3, wobei ein im Vergleich zum Vergleichsverhältnis erhöhtes Verhältnis der Menge an PlGF zur Menge an sFlt-1 in der Testprobe indikativ für ein für die Verabreichung des Aldosteronantagonisten geeignetes Subjekt ist und/oder wobei ein im Vergleich zum Vergleichsverhältnis vermindertes Verhältnis indikativ für ein nicht für die Verabreichung des Aldosteronantagonisten geeignetes Subjekt ist.

5. Verfahren nach einem der Ansprüche 1 bis 4, wobei das Subjekt an Herzinsuffizienz des Stadiums B, C oder D gemäß der ACC/AHA-Klassifikation leidet.

6. Verwendung i) der Biomarker sFlt-1 und PlGF oder ii) eines spezifisch an PlGF bindenden Nachweismittels und eines spezifisch an sFlt-1 bindenden Nachweismittels in einer Blut-, Serum- oder Plasmaprobe eines an Herzinsuffizienz leidenden Subjekts zum Identifizieren eines für die Verabreichung eines Aldosteronantagonisten geeigneten Subjekts.

## Revendications

1. Méthode d'identification d'un sujet éligible à l'administration d'un médicament, ledit médicament étant un antagoniste d'aldostérone, comprenant
a) la détermination de la quantité des biomarqueurs PlGF et sFlt-1 dans un échantillon de sang, de sérum ou de plasma issu d'un sujet souffrant de défaillance cardiaque, et
b1) le calcul d'un rapport de la quantité de PlGF sur la quantité de sFlt-1 (ou vice versa), et la comparaison du rapport calculé à un rapport de référence, par lequel un sujet éligible à l'administration dudit antagoniste d'aldostérone est identifié, ou
b2) la comparaison des quantités déterminées dans l'étape a) à des quantités de référence, par laquelle un sujet éligible à l'administration dudit antagoniste d'aldostérone est identifié.

2. Méthode selon la revendication 1, où l'administration est l'initiation de l'administration de l'antagoniste d'aldostérone, ou l'administration de l'antagoniste d'aldostérone à une dose supérieure.

3. Méthode selon l'une quelconque des revendications 1 ou 2, où le sujet est humain.

4. Méthode selon l'une quelconque des revendications 1 à 3, où un rapport de la quantité de PlGF sur la quantité de sFlt-1 dans l'échantillon d'essai qui est supérieur au rapport de référence est indicatif d'un sujet éligible à l'administration dudit antagoniste d'aldostérone, et/ou où un rapport inférieur au rapport de référence est indicatif d'un sujet qui n'est pas éligible à l'administration dudit antagoniste d'aldostérone.

5. Méthode selon l'une quelconque des revendications 1 à 4, où le sujet souffre de défaillance cardiaque de stade B, C ou D selon la classification de l'ACC/AHA.

6. Utilisation i) des biomarqueurs sFlt-1 et PlGF ou ii) d'un agent de détection se liant spécifiquement à PlGF et d'un agent de détection se liant spécifiquement à sFlt1, dans un échantillon de sang, de sérum ou de plasma d'un sujet souffrant de défaillance cardiaque pour identification d'un sujet éligible à l'administration d'un antagoniste d'aldostérone.
